# EUROPEAN PATENT APPLICATION

(11) **EP 4 556 555 A1**
(43) Date of publication of application: **21.05.2025**
(21) Application number: 23839669.1
(22) Date of filing: 13.07.2023
(51) Int. Cl.: C12N 5/071, C12N 5/10, A61L 27/38, A61L 27/52, A61L 27/58

(54) **FIBRIN GEL SHEET FOR CELL TRANSPLANTATION**

(30) Priority: 14.07.2022 JP 2022113546
(71) Applicant: Orizuru Therapeutics, Inc., Fujisawa-shi Kanagawa 251-8555 (JP)
(72) Inventor: SUGIYAMA Hiroaki, Fujisawa-shi, Kanagawa 251-8555 (JP); UENO Hikaru, Fujisawa-shi, Kanagawa 251-8555 (JP); YOSHIHARA Akifumi, Fujisawa-shi, Kanagawa 251-8555 (JP)
(74) Representative: Jones, Nicholas Andrew
(86) International application number: PCT/JP2023/025809
(87) International publication number: WO 2024/014497

(57) **Abstract**

An object of the present invention is to provide a fibrin gel sheet for cell transplantation that has an unprecedented larger size while cells are uniformly dispersed and embedded, and a method for producing the same. The present invention provides a fibrin gel sheet for cell transplantation, wherein cells are uniformly dispersed and embedded in the fibrin gel sheet, and the fibrin gel sheet has a size of 2.25 cm² or larger in terms of a surface area of one surface and has a thickness of 1 mm or smaller, and a method for producing the same.

## Description

### Technical Field

The present invention relates to a fibrin gel sheet for cell transplantation having an unprecedented large size in which cells are uniformly dispersed and embedded in the fibrin gel sheet, and a method for producing the same.

### [Background Art]

Functional differentiated cells induced from pluripotent stem cells such as induced pluripotent cells or embryonic stem cells (ES cells) are expected as cell sources for transplantation and regenerative medicine. Today, various structures in which such functional differentiated cells are embedded in a biodegradable gel are developed, and research is underway on cell transplantation therapy using these structures.

For example, Non Patent Literature 1 discloses a sheet-shaped structure formed by wrapping pancreatic islet cells embedded in a fibrin gel in a 7 mm × 7 mm Vicryl(R) mesh, and states that the structure was transplanted to a site treated by angiogenesis in advance in a diabetic model rat serving as a recipient.

Non Patent Literature 2 discloses a sheet-shaped structure formed by wrapping pancreatic islet cells embedded in a hydrogel containing polyvinyl alcohol (PVA) in a 20 mm × 15 mm polyethylene terephthalate (PET) mesh, and states that the structure was transplanted to a diabetic model rat serving as a recipient.

Patent Literature 1 discloses a sheet-shaped structure formed by wrapping pancreatic islet cells embedded in a hydrogel containing PVA in a round PET mesh of 15 mm in diameter, and states that the structure was transplanted to a diabetic model mouse serving as a recipient.

### Citation List

### Patent Literature

Patent Literature 1: WO2018/155622

### Non Patent Literature

Non Patent Literature 1: Hirotake Komatsu et al., Transplant International 2020; 33: 806-818
Non Patent Literature 2: Meirigeng Qi et al., Biomaterials. 2004 Dec; 25 (27): 5885-92

### Summary of Invention

### Technical Problem

Meanwhile, previously reported sheet-shaped structures in which functional differentiated cells are embedded in a biodegradable gel are intended for transplantation to diabetic model animals (rats or mice). Their sizes have not been sufficient sizes for targeting large mammals such as humans, and the number of cells to be loaded have not been sufficient, in some times. Hence, there has been a strong demand for the development of such a structure that has a larger size available for humans and the like and comprises more cells in the art.

To solve these problems, the present inventors have attempted to prepare the structure having a larger size using a fibrin gel and consequently found that a larger amount of the fibrin gel prepared for preparing the larger size, when expanded until the desired size to shape a sheet, is difficult to shape into the size of interest due to gelation (solidification) before being sufficiently expanded. The present inventors have also revealed that in the case of shaping a sheet having the desired size while continuously injecting such a large amount of the fibrin gel, cells cause precipitation in a solution to gradually decrease the content of the cells from the start through the end of continuous injection so that the structure having a nonuniform cell density is merely prepared. Such a nonuniform cell density might reduce treatment efficiency or influence long-term engraftment of transplanted cells and is therefore not favorable.

Accordingly, an object of the present invention is to solve these problems found by the present inventors and to provide a sheet-shaped fibrin gel structure (i.e., a fibrin gel sheet) that has an unprecedented larger size while more cells are uniformly dispersed and embedded.

### Solution to Problem

The present inventors have conducted diligent studies to attain the object and consequently found that in the formation of a fibrin gel, the amounts of fibrinogen and thrombin blended and/or a temperature at the time of gelation (solidification) is adjusted, whereby the gelation (solidification) rate of the fibrin gel to be formed can be adjusted, and the fibrin gel can be expanded to shape a fibrin gel sheet having a desired larger size.

The present inventors have also found that in the formation of a fibrin gel, biodegradable particles are added and suspended in a fibrinogen solution of cells suspended therein, or a biodegradable gelator is added and dissolved therein, whereby the cells are prevented from being precipitated in the fibrinogen solution so that a uniformly dispersed and suspended state of the cells can be maintained; and the solution is allowed to gelate (solidified), whereby a fibrin gel sheet having a uniform cell density can be obtained even in shaping through continuous injection.

The present invention is based on these novel findings and encompasses the following inventions.
[1] A fibrin gel sheet for cell transplantation, wherein cells are uniformly dispersed and embedded in the fibrin gel sheet, and the fibrin gel sheet has a size of 2.25 cm² or larger in terms of a surface area of one surface and has a thickness of 1 mm or smaller.
[2] The fibrin gel sheet according to [1], wherein the cells are embedded in an amount of more than 1.5 × 10⁶ cells/cm².
[3] The fibrin gel sheet according to [1] or [2], wherein the cells are in a spheroid form.
[4] The fibrin gel sheet according to any of [1] to [3], wherein the cells are iPS cell-derived pancreatic islet cells.
[5] The fibrin gel sheet according to any of [1] to [4], further comprising a biodegradable gelator.
[6] The fibrin gel sheet according to [5], wherein the fibrin gel sheet comprises the biodegradable gelator in an amount of 0.2 to 2 w/v%.
[7] The fibrin gel sheet according to [5] or [6], wherein the biodegradable gelator is collagen.
[8] The fibrin gel sheet according to any of [1] to [4], wherein biodegradable particles are further embedded.
[9] The fibrin gel sheet according to [8], wherein the biodegradable particles have a size with which the biodegradable particles are capable of passing through an opening size of 100 µm to 1000 µm.
[10] The fibrin gel sheet according to [8] or [9], wherein the fibrin gel sheet comprises the biodegradable particles in an amount of 10 to 30 w/v%.
[11] The fibrin gel sheet according to any of [8] to [10], wherein the biodegradable particles are gelatin gel particles.
[12] The fibrin gel sheet according to any of [1] to [11], further comprising a support.
[13] A method for producing a fibrin gel sheet for cell transplantation in which cells are uniformly dispersed and embedded in the fibrin gel sheet, the fibrin gel sheet having a size of 2.25 cm² or larger in terms of a surface area of one surface and having a thickness of 1 mm or smaller, the method comprising the step of
   allowing thrombin to act on a fibrinogen solution of the cells suspended therein, and shaping a sheet that has a size of 2.25 cm² or larger in terms of a surface area of one surface and has a thickness of 1 mm or smaller, followed by gelation.
[14] The production method according to [13], wherein the cells are suspended in the fibrinogen solution in an amount that attains more than 1.5 × 10⁶ cells/cm² in the fibrin gel sheet.
[15] The production method according to [13] or [14], wherein the cells are in a spheroid form.
[16] The production method according to any of [13] to [15], wherein the cells are iPS cell-derived pancreatic islet cells.
[17] The production method according to any of [13] to [16], wherein the fibrinogen solution of the cells suspended therein further comprises a biodegradable gelator.
[18] The production method according to [17], wherein the fibrinogen solution of the cells suspended therein comprises the biodegradable gelator in an amount of 0.2 to 2 w/v%.
[19] The production method according to [17] or [18], wherein the biodegradable gelator is collagen.
[20] The production method according to any of [17] to [19], wherein the sheet is shaped by applying the solution onto a support.
[21] The production method according to [20], wherein the solution is applied onto the support by bioprint.
[22] The production method according to [20] or [21], wherein the support comprises the thrombin.
[23] The production method according to any of [13] to [16], wherein the fibrinogen solution of the cells suspended therein further comprises biodegradable particles.
[24] The production method according to [23], wherein the biodegradable particles have a size with which the biodegradable particles are capable of passing through an opening size of 100 µm to 1000 µm.
[25] The production method according to [23] or [24], wherein the fibrinogen solution of the cells suspended therein comprises the biodegradable particles in an amount of 10 to 30 w/v%.
[26] The production method according to any of [23] to [25], wherein the biodegradable particles are gelatin gel particles.
[27] The production method according to any of [23] to [26], wherein the sheet is shaped by applying the solution onto a support.
[28] The production method according to [27], wherein the solution is applied onto the support by bioprint.
[29] The production method according to [27] or [28], wherein the support comprises the thrombin.
[30] The production method according to any of [13] to [16], wherein the thrombin is allowed to act in an amount that attains a blending ratio of 0.4 U or less to 1 mg of fibrinogen.
[31] The production method according to [30], wherein the sheet is shaped under cooling at 2 to 8°C.
[32] The production method according to [30] or [31], further comprising the step of integrating the support with the fibrin gel sheet.
[33] A fibrin gel sheet laminate for cell transplantation, wherein a plurality of fibrin gel sheets according to any of [1] to [12] are laminated.

[1a] A fibrin gel sheet for use in a cell transplantation therapy method, wherein cells are uniformly dispersed and embedded in the fibrin gel sheet, and the fibrin gel sheet has a size of 2.25 cm² or larger in terms of a surface area of one surface and has a thickness of 1 mm or smaller
[2a] The fibrin gel sheet according to [1a], wherein the cells are embedded in an amount of more than 1.5 × 10⁶ cells/cm².
[3a] The fibrin gel sheet according to [1a] or [2a], wherein the cells are in a spheroid form.
[4a] The fibrin gel sheet according to any of [1a] to [3a], wherein the cells are iPS cell-derived pancreatic islet cells.
[5a] The fibrin gel sheet according to any of [1a] to [4a], further comprising a biodegradable gelator.
[6a] The fibrin gel sheet according to [5a], wherein the fibrin gel sheet comprises the biodegradable gelator in an amount of 0.2 to 2 w/v%.
[7a] The fibrin gel sheet according to [5a] or [6a], wherein the biodegradable gelator is collagen.
[8a] The fibrin gel sheet according to any of [1a] to [4a], further comprising biodegradable particles.
[9a] The fibrin gel sheet according to [8a], wherein the biodegradable particles have a size with which the biodegradable particles are capable of passing through an opening size of 100 µm to 1000 µm.
[10a] The fibrin gel sheet according to [8a] or [9a], wherein the fibrin gel sheet comprises the biodegradable particles in an amount of 10 to 30 w/v%.
[11a] The fibrin gel sheet according to any of [8a] to [10a], wherein the biodegradable particles are gelatin gel particles.
[12a] The fibrin gel sheet according to any of [1a] to [10a], further comprising a support.
[33a] A fibrin gel sheet laminate for use in cell transplantation therapy method, wherein a plurality of fibrin gel sheets according to any of [1a] to [12a] are laminated.

The present specification encompasses the contents described in the specification and/or drawings of Japanese Patent Application No. 2022-113546 filed on July 14, 2022 on which the priority of the present application is based.

All publications, patents and patent applications cited herein are incorporated herein by reference in their entirety.

### Advantageous Effects of Invention

The present invention can provide a fibrin gel sheet that has an unprecedented larger size while more cells are uniformly dispersed and embedded, and a method for producing the same.

### Brief Description of Drawings

[Figure 1] Figure 1 is a photographic view showing the appearance of (A) an iPIC-embedded fibrin gel mass and (B) an iPIC-embedded fibrin gel sheet. Scale bar: 10 mm.
[Figure 2] Figure 2 is a graph chart showing analysis results of measuring a human c-peptide concentration in blood over time by ELISA in a nude rat in which an iPIC-embedded fibrin gel mass or an iPIC-embedded fibrin gel sheet was subcutaneously transplanted.
[Figure 3] Figure 3 is a photographic view showing histological analysis results about a transplantation site 10 days and 6 weeks after transplantation in a nude rat in which (A) an iPIC-embedded fibrin gel mass or (B) an iPIC-embedded fibrin gel sheet was subcutaneously transplanted. Scale bar: 1 mm.
[Figure 4] Figure 4 is a graph chart showing results of analyzing the relationship between a thrombin concentration and a gelation time of a fibrin gel.
[Figure 5] Figure 5 is photographic view showing the appearance of a large animal/human-sized iPIC-embedded fibrin gel sheet (A), which was produced by a method using a cooled metal mold, and results of observing positions of 1 to 4 in the sheet under a phase-contrast microscope (B).
[Figure 6] Figure 6 is a photographic view showing the state of iPIC after being suspended in (A) a fibrinogen solution and (B) a fibrinogen + gelatin microsphere solution and left standing for a predetermined time. The arrowhead in (A) depicts the precipitation of iPIC.
[Figure 7] Figure 7 is a graph chart showing analysis results of measuring a human c-peptide concentration in blood over time by ELISA in a nude rat in which an iPIC-embedded fibrin gel sheet or an iPIC-embedded fibrin + gelatin microsphere gel sheet was subcutaneously transplanted.
[Figure 8] Figure 8 is a photographic view showing the appearance and cross section of a large animal/human-sized iPIC-embedded fibrin + gelatin microsphere gel sheet prepared manually by pipette injection or using a 3D printer (A), shows results of image-analyzing a luminance distribution of each sheet and calculating standard deviation (B), and is a graph chart showing results of measuring the number of cells contained in any four locations (1 to 4) of the fibrin + gelatin microsphere gel sheet prepared using a 3D printer (C).
[Figure 9] Figure 9 is a graph chart showing results of measuring the number (concentration) of cells in a fibrinogen + collagen solution ejected from a syringe.
[Figure 10] Figure 10 is a graph chart showing analysis results of measuring a human c-peptide concentration in blood by ELISA in a nude rat in which an iPIC-embedded fibrin + gelatin microsphere gel sheet or an iPIC-embedded fibrin + collagen gel sheet was subcutaneously transplanted.
[Figure 11] Figure 11 is a graph chart showing results of measuring the number of cells contained in any six locations (1 to 6) of a large animal/human-sized iPIC-embedded fibrin + collagen gel sheet prepared using a 3D printer.

### Description of Embodiments

### 1. Terminology

Hereinafter, the terms described herein will be described.

As used herein, "about" refers to a value which may vary up to plus or minus 25%, 20%, 10%, 8%, 6%, 5%, 4%, 3%, 2%, or 1% from the reference value. Preferably, the term "about" refers to a range from minus or plus 15%, 10%, 5%, or 1% from the reference value.

As used herein, "comprise(s)" or "comprising" means inclusion of the element(s) following the word without limitation thereto. Accordingly, it indicates inclusion of the element(s) following the word, but does not indicate exclusion of any other element.

As used herein, "consist(s) of" or "consisting of" means inclusion of all the element(s) following the phrase and limitation thereto. Accordingly, the phrase "consist(s) of" or "consisting of" indicates that the enumerated element(s) is required or essential and substantially no other elements exist.

As used herein, "without the use of feeder cell(s)" means basically containing no feeder cells and using no medium preconditioned by culturing feeder cells. Accordingly, the medium does not contain any substance, such as a growth factor or a cytokine, secreted by feeder cells.

"Feeder cells" or "feeder" means cells that are co-cultured with another kind of cells, support the cells, and provide an environment that allows the cells to grow. The feeder cells may be derived from the same species as or a different species from the cells that they support. For example, as a feeder for human cells, human skin fibroblasts or human embryonic-stem cells may be used or a primary culture of murine embryonic fibroblasts or immortalized murine embryonic fibroblasts may be used. The feeder cells can be inactivated by exposure to radiation or treatment with mitomycin C.

As used herein, "adhered (adherent)" refers to cells are attached to a container, for example, cells are attached to a cell culture dish or a flask made of a sterilized plastic (or coated plastic) in the presence of an appropriate medium. Some cells cannot be maintained or grow in culture without adhering to the cell culture container. In contrast, non-adherent cells can be maintained and proliferate in culture without adhering to the container.

As used herein, "culture" refers to maintaining, growing, and/or differentiating cells in in vitro environment. "Culturing" means maintaining, proliferating, and/or differentiating cells out of tissue or the living body, for example, in a cell culture dish or flask. The culture includes two-dimensional culture (plane culture) and three-dimensional culture (suspension culture).

As used herein, "purify(ies)" and "purification" refer to removing impurities in a composition such as a composition of cells and making it pure for a certain component and "purified" refers, when used to describe a composition of cells, to a composition of cells in which the amount of impurities is decreased in comparison with the percentage of such components in the composition of cells before purification and the purity of a certain component is improved. For example, a composition of cells can be purified for a target cell type and, accordingly, the percentage of the target cell type is increased in comparison with the percentage of the target cells present in the composition of cells before the purification. A composition of cells can be purified for a target cell type by a method of selecting and sorting cells known in the art. A composition of cells can be purified by a specific process of sorting or selection described herein. In a certain embodiment of the present invention, the purity of a target cell is brought by a method of purifying a target cell to at least 70%, 80%, 85%, 90%, 95%, 97%, 98%, or 99% or to the extent at which impurities (including contaminant cells) are undetectable.

As used herein, "growth factors" are endogenous proteins that promote differentiation and/or proliferation of particular cells. Examples of "growth factors" include epidermal growth factor (EGF), acid fibroblast growth factor (aFGF), basic fibroblast growth factor (bFGF), hepatocyte growth factor (HGF), insulin-like growth factor 1 (IGF-1), insulin-like growth factor 2 (IGF-2), keratinocyte growth factor (KGF), nerve growth factor (NGF), platelet-derived growth factor (PDGF), transformation growth factor beta (TGF-β), vascular endothelial growth factor (VEGF), transferrin, various interleukins (for example, IL-1 to IL-18), various colony stimulating factors (for example, granulocyte/macrophage-colony stimulating factor (GM-CSF)), various interferons (for example, IFN-y, and the like), and other cytokines having effects on stem cells, for example, stem cell factor (SCF), and erythropoietin (Epo).

As used herein, "ROCK inhibitors" means substances that inhibit Rho kinase (ROCK: Rho-associated, coiled-coil containing protein kinase) and may be substances that inhibit either of ROCK I and ROCK II. The ROCK inhibitors are not particularly limited as long as they have the aforementioned function and examples include N-(4-pyridinyl)-4β-[(R)-1-aminoethyl]cyclohexane-1α-carboxamide (that may be herein also referred to as Y27632), Fasudil (HA1077), (2S)-2-methyl-1-[(4-methyl-5-isoquinolinyl)sulfonyl]hexahydro-1H-1,4-diazepine (H-1152), 4β-[(1R)-1-aminoethyl]-N-(4-pyridyl)benzene-1α-carbamide (Wf-536), N-(1H-pyrrolo[2,3-b]pyridin-4-yl)-4PER[(R)-1-aminoethyl]cyclohexane-1α-carboxamide (Y-30141), N-(3-{[2-(4-amino-1,2,5-oxadiazol-3-yl)-1-ethyl-1H-imidazo[4,5-c]pyridin-6-yl]oxy}phenyl)-4-{[2-(4-morpholinyl)ethyl]-oxy}benzamide (GSK269962A), N-(6-fluoro-1H-indazol-5-yl)-6-methyl-2-oxo-4-[4-(trifluoromethyl)phenyl]-3,4-dihydro-1H-pyridine-5-carboxamide (GSK429286A). The ROCK inhibitors are not limited to these and antisense oligonucleotides and siRNA to ROCK mRNA, antibodies that bind to ROCK, and dominant negative ROCK mutants can also be used as ROCK inhibitors, commercially available, or synthesized according to a known method.

As used herein, "GSK3β inhibitors" are substances having the inhibitory activity for GSK3β (glycogen synthase kinase 3β). GSK3 (glycogen synthase kinase 3) is a serine/threonine protein kinase and involved in many signaling pathways associated with the production of glycogen, apoptosis, maintenance of stem cells, etc. GSK3 has the 2 isoforms α and β. "GSK3β inhibitors" used in the present invention are not particularly limited as long as they have the GSK3β-inhibiting activity and they may be substances having both the GSK3α-inhibiting activity and the GSK3β-inhibiting activity.

Examples of GSK3β inhibitors include CHIR98014 (2-[[2-[(5-nitro-6-aminopyridin-2-yl)amino]ethyl]amino]-4-(2,4-dichlorophenyl)-5-(1H-imidazol-1-yl)pyrimidine), CHIR99021 (6-[[2-[[4-(2,4-dichlorophenyl)-5-(4-methyl-1H-imidazol-2-yl)-2-pyrimidinyl]amino]ethyl]amino]nicotinonitrile), TDZD-8 (4-benzyl-2-methyl-1,2,4-thiadiazolidine-3,5-dione), SB216763 (3-(2,4-dichlorophenyl)-4-(1-methyl-1H-indol-3-yl)-1H-pyrrole-2,5-dione), TWS-119 (3-[6-(3-aminophenyl)-7H-pyrrolo[2,3-d]pyrimidin-4-yloxy]phenol), kenpaullone, 1-azakenpaullone, SB216763 (3-(2,4-dichlorophenyl)-4-(1-methyl-1H-indol-3-yl)-1H-pyrrole-2,5-dione), SB415286 (3-[(3-chloro-4-hydroxyphenyl)amino]-4-(2-nitrophenyl)-1H-pyrrole-2,5-dione), and AR-AO144-18, CT99021, CT20026, BIO, BIO-acetoxime, pyridocarbazole-ruthenium cyclopentadienyl complex, OTDZT, alpha-4-dibromoacetophenone, lithium, and the like. GSK3β inhibitors are not limited to these and antisense oligonucleotides and siRNA to GSK3β mRNA, antibodies that bind to GSK3β, dominant negative GSK3β mutants, and the like can also be used as GSK3β inhibitors, commercially available, or synthesized according to a known method.

As used herein, examples of "serum replacement" include KnockOut(TM) Serum Replacement (KSR: Thermo Fisher Scientific), StemSure(R) Serum Replacement (Wako), B-27 supplement, N2-supplement, albumin (for example, lipid rich albumin), insulin, transferrin, fatty acids, collagen precursors, trace elements (for example, zinc, selenium (for example, sodium selenite)), 2-mercaptoethanol, 3'-thiolglycerol, or mixtures thereof (for example, ITS-G). Preferred serum replacements are B-27 supplement, KSR, StemSure(R) Serum Replacement, ITS-G. The concentration of serum replacement in a medium when added into a medium is 0.01-10% by weight, and preferably 0.1-2% by weight. In the present invention, "serum replacement" is preferably used instead of serum.

As used herein, "factor having CDK8/19-inhibiting activity" means any substance having the inhibitory activity for CDK8/19. CDK8, in contrast to the other proteins of the same CDK family, is not required for cell proliferation. The inhibition of CDK8 has no great effect under usual conditions. CDK19 and CDK8 are similar to each other. Usually, the inhibition of CDK8 also involves the inhibition of CDK19. Conventionally known "factor having CDK8/19-inhibiting activity" can be used, and such a CDK8/19 inhibitor can be found from patent literatures or non patent literatures. For example, among compounds described in US2012/0071477, WO2015/159937, WO2015/159938, WO2013/116786, WO2014/0038958, WO2014/134169, JP2015/506376, US2015/0274726, US2016/0000787, WO2016/009076, WO2016/0016951, WO2016/018511, WO2016/100782 and WO2016/182904, a compound or a salt thereof having CDK8/19-inhibiting activity can be used as "factor having CDK8/19-inhibiting activity" according to the present invention. In the present invention, for example, diethyl (E)-(4-(3-(5-(4-fluorophenyl)-1-methyl-1H-pyrazol-4-yl)acrylamido)benzyl)phosphonate, 2-(4-(4-(isoquinolin-4-yl)phenyl)-1H-pyrazol-1-yl)-N,N-dimethylacetamide, 4-((2-(6-(4-methylpiperazine-1-carbonyl)naphthalen-2-yl)ethyl)amino)quinazoline-6-carbonitrile, 4-(4-(2,3-dihydrobenzo[b][1,4]dioxin-6-yl)-1H-pyrazol-3-yl)benzene-1,3-diol, 3-(2-(imidazo[1,2-b]pyridazin-6-ylthio)ethyl)-4-(naphthalen-1-ylsulfornyl)-3,4-dihydroquinoxalin-2(1H)-one, and (E)-3-(4-(1-cyclopropyl-1H-pyrazol-4-yl)pyridin-3-yl)-N-(4-(morpholinomethyl)phenyl)acrylamide or salts thereof can be used as "factor having CDK8/19-inhibiting activity". The factor having CDK8/19-inhibiting activity is not limited to these compounds, and an antisense oligonucleotide or siRNA against CDK8/19 mRNA, an antibody binding to CDK8/19, a dominant negative CDK8/19 mutant, or the like can also be used as the factor having CDK8/19-inhibiting activity, commercially available, or synthesized according to a known method.

### 2. Fibrin gel sheet for cell transplantation

The present invention relates to a fibrin gel sheet for cell transplantation, wherein cells are uniformly dispersed and embedded in the fibrin gel sheet (hereinafter, also simply referred to as "fibrin gel sheet of the present invention").

A feature of the fibrin gel sheet of the present invention is to have a size that cannot be achieved by a conventional fibrin gel sheet for cell transplantation. The surface area of one surface (that is, any of the major surfaces of the sheet or a surface on one side of the sheet) of the sheet is 2.25 cm² or larger, preferably 4 cm² or larger, more preferably 9 cm² or larger, further preferably 16 cm² or larger, still further preferably 25 cm² or larger, especially preferably 36 cm² or larger, especially more preferably 49 cm² or larger, especially further preferably 64 cm² or larger, especially still further preferably 81 cm² or larger, particularly preferably 100 cm² or larger. The upper limit thereof is not particularly limited and may be appropriately selected depending on the size or shape of a transplantation site. The fibrin gel sheet can have a size of, for example, 400 cm² or smaller.

The range of the surface area in the fibrin gel sheet of the present invention can be expressed using two numeric values respectively selected from the numeric values of the lower limit and the upper limit. The surface area of the fibrin gel sheet of the present invention can be a size appropriately selected from the range of, for example, 2.25 cm² or larger to 400 cm² or smaller, preferably 4 cm² or larger to 400 cm² or smaller, more preferably 9 cm² or larger to 400 cm² or smaller, further preferably 16 cm² or larger to 400 cm² or smaller, still further preferably 25 cm² or larger to 400 cm² or smaller, especially preferably 36 cm² or larger to 400 cm² or smaller, especially more preferably 49 cm² or larger to 400 cm² or smaller, especially further preferably 64 cm² or larger to 400 cm² or smaller, especially still further preferably 81 cm² or larger to 400 cm² or smaller, particularly preferably 100 cm² or larger to 400 cm² or smaller.

The shape of the one surface of the fibrin gel sheet of the present invention is not particularly limited and may be appropriately selected depending on the size or shape of a transplantation site. Examples thereof include, but are not limited to, polygons (for example, triangles, tetragons, pentagons, hexagons, and octagons), rounded polygons, round shapes, and oval shapes.

When the fibrin gel sheet of the present invention has, for example, a tetragon, the size has a length of 15 mm or larger, preferably 20 mm or larger, more preferably 30 mm or larger, further preferably 40 mm or larger, still further preferably 50 mm or larger, especially preferably 60 mm or larger, especially more preferably 70 mm or larger, especially further preferably 80 mm or larger, especially still further preferably 90 mm or larger, particularly preferably 100 mm or larger. The upper limit thereof is not particularly limited and can be, for example, 200 mm or smaller. The fibrin gel sheet of the present invention has a width of 15 mm or larger, preferably 20 mm or larger, more preferably 30 mm or larger, further preferably 40 mm or larger, still further preferably 50 mm or larger, especially preferably 60 mm or larger, especially more preferably 70 mm or larger, especially further preferably 80 mm or larger, especially still further preferably 90 mm or larger, particularly preferably 100 mm or larger. The upper limit thereof is not particularly limited and can be, for example, 200 mm or smaller.

The range of the length and the width can be expressed using two numeric values respectively selected from the numeric values of the lower limit and the upper limit. The size (length × width (which may be width × length)) of the fibrin gel sheet of the present invention can be a size appropriately selected from the range of, for example, 15 mm or larger to 200 mm or smaller × 15 mm or larger to 200 mm or smaller, preferably 20 mm or larger to 200 mm or smaller × 20 mm or larger to 200 mm or smaller, more preferably 30 mm or larger to 200 mm or smaller × 30 mm or larger to 200 mm or smaller, further preferably 40 mm or larger to 200 mm or smaller × 40 mm or larger to 200 mm or smaller, still further preferably 50 mm or larger to 200 mm or smaller × 50 mm or larger to 200 mm or smaller, especially preferably 60 mm or larger to 200 mm or smaller × 60 mm or larger to 200 mm or smaller, especially more preferably 70 mm or larger to 200 mm or smaller × 70 mm or larger to 200 mm or smaller, especially further preferably 80 mm or larger to 200 mm or smaller × 80 mm or larger to 200 mm or smaller, especially still further preferably 90 mm or larger to 200 mm or smaller × 90 mm or larger to 200 mm or smaller, particularly preferably 100 mm or larger to 200 mm or smaller × 100 mm or larger to 200 mm or smaller. Examples of the size (length × width (which may be width × length)) of the fibrin gel sheet of the present invention include, but are not limited to, 15 mm or larger × 15 mm or larger, preferably 20 mm × 20 mm, 20 mm × 30 mm, or 30 mm × 30 mm, more preferably 40 mm × 40 mm, 40 mm × 50 mm, 50 mm × 50 mm, 40 mm × 60 mm, or 60 mm × 60 mm, further preferably 70 mm × 70 mm, 80 mm × 80 mm, 90 mm × 90 mm, 100 mm × 100 mm, or 200 mm × 200 mm.

The thickness of the fibrin gel sheet of the present invention can be appropriately set depending on a factor such as the size or amount of the cells to be embedded or the size of a transplantation site and is preferably 1 mm or smaller. When the thickness of the fibrin gel sheet of the present invention is 1 mm or smaller, the supply of a nutrient or oxygen to the cells embedded in the fibrin gel sheet, for example, can be efficiently performed and rapid degradation of the fibrin gel after transplantation can be achieved, which are preferred. The thickness of the fibrin gel sheet of the present invention can be, for example, 1 mm or smaller, 900 µm or smaller, 800 µm or smaller, 700 µm or smaller, 600 µm or smaller, 500 µm or smaller, or 400 µm or smaller. The lower limit thereof is not particularly limited and can be, for example, 100 µm or larger, 200 µm or larger, or 300 µm or larger. The range of the thickness of the fibrin gel sheet of the present invention can be expressed using two numeric values respectively selected from the numeric values of the upper limit and the lower limit. The thickness of the fibrin gel sheet of the present invention can be, for example, 100 µm or larger to 1 mm or smaller, 100 µm to 800 µm or smaller, 200 µm to 600 µm or smaller, or 300 µm or larger to 400 µm or smaller and can be a thickness appropriately selected from these ranges. The thickness of the fibrin gel sheet of the present invention is, for example, about 400 µm. The thickness of the fibrin gel sheet of the present invention does not have to be uniform throughout the sheet, and the thickest part can fall within the range described above.

As used herein, "uniformly dispersed and embedded" as to the cells means that the inoculated cells are contained so as to be widely distributed in the fibrin gel sheet of the present invention without being localized to any site of the fibrin gel sheet. It is not required that all the cells should be wholly buried in the fibrin gel sheet. However, cultures of cells inoculated to the surface of the fibrin gel and adherent-cultured are different from the form in which the cells are "dispersed and embedded" because the cells are localized only to the surface of the fibrin gel sheet. More specifically, as used herein, "uniformly dispersed and embedded" as to the cells means that the respective numbers of cells (cell densities) contained in predetermined ranges have small bias among any two or more locations (for example, two locations, four locations, or six locations) in the fibrin gel sheet of the present invention, and preferably means that the values of the respective numbers of cells fall within the range of within ±20%, preferably within ±15%, more preferably within ±10%, of an average value thereof. As used herein, "uniformly dispersed and embedded" as to the cells means the positional relationship among the inoculated cells. For example, the case in which cells inoculated to the surface or inside of the fibrin gel proliferate by culture so that the cell groups of the inoculated cells differing in origin are contacted/bound with each other does not correspond to the "uniformly dispersed and embedded" state of the cells because the cells are at least not dispersed among the inoculated cells. Examples of such a contacted/bound state among the inoculated cells include cell cultures (for example, cell sheets) formed by inoculation and culture on the surface or in the inside of the fibrin gel. Such a form is not intended to be included in the present invention. On the other hand, unless such inoculated cells are in the contacted/bound state, for example, the state of a cell mass or a cell aggregate in which the cells gather or aggregate with each other, that is, a spheroid form, or proliferation of the inoculated cells is not intended to be excluded from the form of the inoculated cells.

In the present invention, "cells" are cells for cell therapy that are inoculated to a patient in need thereof, and can be appropriately selected depending on symptoms of the patient. Examples thereof include cells having functions equivalent or similar to cells constituting organs or tissues of, but not limited to, the epidermis, the nerve, the brain, the spinal cord, the esophagus, the stomach, the small intestine, the large intestine, the urinary bladder, the urethra, the lung, the thyroid gland, the pancreas, the liver, muscle, skeleton, the heart, vascular vessel, the spleen, the kidney, and blood, or precursor cells thereof. Any of "cells" may be used singly, or different cells may be used in combination. The form of the cells in the fibrin gel sheet of the present invention is not particularly limited and may be mutually separated cells (single-cell state), may be in the state of a cell mass or a cell aggregate in which the cells gather or aggregate with each other, that is, a spheroid form, or may be suspension-cultured cells. Examples of the cells in a spheroid form include, but are not limited to, pancreatic islet cells, hepatocytes, and cardiomyocytes.

In the present invention, "cells" may be cells collected from the living body, may be cultured cells, and may be frozen and thawed cells of these cells. Preferably, in the present invention, "cells" are pluripotent stem cell-derived cells obtained by inducing the differentiation of pluripotent stem cells.

As used herein, "pluripotency" means the ability to differentiate into tissues and cells having various different shapes and functions and to differentiate into cells of any lineage of the 3 germ layers. "Pluripotency" is different from "totipotency", which is the ability to differentiate into any tissue of the living body, including the placenta, in that pluripotent cells cannot differentiate into the placenta and therefore, do not have the ability to form an individual.

As used herein, "multipotency" means the ability to differentiate into plural and limited numbers of linages of cells. For example, mesenchymal stem cells, hematopoietic stem cells, neural stem cells are multipotent, but not pluripotent.

As used herein, "pluripotent stem cells" refers to embryonic-stem cells (ES cells) and cells potentially having a pluripotency similar to that of ES cells, that is, the ability to differentiate into various tissues (all of the endodermal, mesodermal, and ectodermal tissues) in the living body. Examples of cells having a pluripotency similar to that of ES cells include "induced pluripotent stem cells" (that may be herein also referred to as "iPS cells"). In the present invention, preferably, pluripotent stem cells are human pluripotent stem cells.

Available "ES cells" include murine ES cells, such as various murine ES cell lines established by inGenious, Institute of Physical and Chemical Research (RIKEN), and the like, and human ES cells, such as various human ES cell lines established by National Institutes of Health (NIH), RIKEN, Kyoto University, Cellartis, and the like. For example, available ES cell lines include CHB-1 to CHB-12, RUES1, RUES2, HUES1 to HUES28 from NIH, and the like; H1 and H9 from WiCell Research Institute; and KhES-1, KhES-2, KhES-3, KhES-4, KhES-5, SSES1, SSES2, SSES3 from RIKEN, and the like.

"Induced pluripotent stem cells" refers to cells that are obtained by reprograming mammalian somatic cells or undifferentiated stem cells by introducing particular factors (nuclear reprogramming factors). At present, there are various "induced pluripotent stem cells" and iPS cells established by Yamanaka, et al. by introducing the 4 factors Oct3/4, Sox2, Klf4, and c-Myc into murine fibroblasts (Takahashi K, Yamanaka S., Cell, (2006) 126: 663-676); iPS cells derived from human cells, established by introducing similar 4 factors into human fibroblasts (Takahashi K, Yamanaka S., et al. Cell, (2007) 131: 861-872.); Nanog-iPS cells established by sorting cells using expression of Nanog as an indicator after introduction of the 4 factors (Okita, K., Ichisaka, T., and Yamanaka, S. (2007). Nature 448, 313-317.); iPS cells produced by a method not using c-Myc (Nakagawa M, Yamanaka S., et al. Nature Biotechnology, (2008) 26, 101-106); and iPS cells established by introducing 6 factors in a virus-free way (Okita K et al. Nat. Methods 2011 May; 8(5): 409-12, Okita K et al. Stem Cells. 31 (3) 458-66) may be also used. Also, induced pluripotent stem cells established by introducing the 4 factors OCT3/4, SOX2, NANOG, and LIN28 by Thomson et al. (Yu J., Thomson JA. et al., Science (2007) 318: 1917-1920.); induced pluripotent stem cells produced by Daley et al. (Park IH, Daley GQ. et al., Nature (2007) 451: 141-146); induced pluripotent stem cells produced by Sakurada et al. (Japanese Unexamined Patent Application Publication No. 2008-307007) and the like may be used.

In addition, any of known induced pluripotent stem cells known in the art described in all published articles (for example, Shi Y., Ding S., et al., Cell Stem Cell, (2008) Vol 3, Issue 5, 568-574; Kim JB., Scholer HR., et al., Nature, (2008) 454, 646-650; Huangfu D., Melton, DA., et al., Nature Biotechnology, (2008) 26, No. 7, 795-797) or patents (for example, Japanese Unexamined Patent Application Publication No. 2008-307007, Japanese Unexamined Patent Application Publication No. 2008-283972, US2008-2336610, US2009-047263, WO2007-069666, WO2008-118220, WO2008-124133, WO2008-151058, WO2009-006930, WO2009-006997, WO2009-007852) may be used.

Available induced pluripotent cell lines include various iPS cell lines established by NIH, Institute of Physical and Chemical Research (RIKEN), Kyoto University and the like. For example, such human iPS cell lines include the RIKEN cell lines HiPS-RIKEN-1A, HiPS-RIKEN-2A, HiPS-RIKEN-12A, and Nips-B2 and the Kyoto University cell lines Ff-WJ-18, Ff-I01s01, Ff-I01s02, Ff-I01s04, Ff-I01s06, Ff-I14s03, Ff-I14s04, QHJI01s01, QHJI01s04, QHJI14s03, QHJI14s04, 253G1, 201B7, 409B2, 454E2, 606A1, 610B1, 648A1, CDI cell lines MyCell iPS Cells (21525.102.10A), MyCell iPS Cells (21526.101.10A), and the like.

Examples of the pluripotent stem cell-derived cells that may be used in the present invention include, but are not limited to, iPS cell-derived pancreatic islet cells (hereinafter, also referred to as "iPIC"). The iPIC includes insulin-producing cells and/or pancreatic β cells. The insulin-producing cells are cells characterized by the expression of at least one of the markers insulin and NK6 homeobox 1 (NKX6.1). The pancreatic β cells are cells more mature than the insulin-producing cells and are characterized by the expression of at least one of the markers MAFA, UCN3, and IAPP. "Marker" means a cell antigen or a gene thereof that is specifically expressed depending on a predetermined cell type, such as "marker protein" and "marker gene". Preferably, a marker is a cell surface marker and this allows concentration, isolation, and/or detection of living cells. A marker can be a positive selection marker or a negative selection marker.

The detection of a marker protein can be conducted by an immunological assay (for example, ELISA, immunostaining, or flow cytometry) using an antibody specific for the marker protein. The detection of a marker gene can be conducted by a method of amplifying and/or detecting nucleic acid known in the art (RT-PCR, microarray, biochip, or the like). For example, "positive" for a marker protein means being detected to be positive by flow cytometry and "negative" therefor means being equal to or less than the lower detection limit in flow cytometry. Also, "positive" for a marker gene means being detected by RT-PCR and "negative" therefor means being equal to or less than the lower detection limit in RT-PCR.

"Expression" is defined as transcription and/or translation of a certain nucleotide sequence driven by an intracellular promoter.

The iPIC can be obtained by inducing the differentiation of pluripotent stem cells in accordance with a known approach (WO2009/012428; WO2016/021734; and Stem Cell Research (2015) 14, 185-197)). Specifically, the iPIC can be obtained using the following steps of induction of differentiation:
step 1) inducing the differentiation of pluripotent stem cells into definitive endoderm cells;
step 2) inducing the differentiation of the definitive endoderm cells into primitive gut tube cells;
step 3) inducing the differentiation of the primitive gut tube cells into posterior foregut cells;
step 4) inducing the differentiation of the posterior foregut cells into pancreatic progenitor cells;
step 5) inducing the differentiation of the pancreatic progenitor cells into endocrine progenitor cells; and
step 6) inducing the differentiation of the endocrine progenitor cells into iPIC.

Hereinafter, each step will be described, though the induction of differentiation into each cell is not limited by these approaches.

### Step 1) Differentiation into definitive endoderm cells

The pluripotent stem cells are first allowed to differentiate into definitive endoderm cells. Methods for inducing the definitive endoderm from pluripotent stem cells have already been known, and any of the methods may be used. Preferably, the pluripotent stem cells are cultured in a medium containing activin A, more preferably a medium containing activin A, a ROCK inhibitor, and a GSK3β inhibitor, to thereby differentiate into definitive endoderm cells. The number of cells at the start of culture is not particularly limited and is 22000 to 150000 cells/cm², preferably 22000 to 100000 cells/cm², more preferably 22000 to 80000 cells/cm². The culture period is 1 day to 4 days, preferably 1 day to 3 days, particularly preferably 3 days.

The culture temperature is not particularly limited, and the culture is performed at 30 to 40°C (for example, 37°C). The concentration of carbon dioxide in a culture container is on the order of, for example, 5%. The culture may be performed by any of two-dimensional culture and three-dimensional culture.

The medium used in this step may be a basal medium for use in the culture of mammalian cells, such as RPMI 1640 medium, MEM medium, iMEM medium, DMEM/F12 medium, Improved MEM Zinc Option medium, Improved MEM/1% B-27/Penicillin Streptomycin medium, or MCDB131/20 mM Glucose/NaHCO₃/FAF-BSA/ITS-X/GlutaMAX(TM)/ascorbic acid/Penicillin Streptomycin medium.

The concentration of the activin A in the medium is usually 30 to 200 ng/mL, preferably 50 to 150 ng/mL, more preferably 70 to 120 ng/mL, particularly preferably about 100 ng/mL.

In another embodiment, the activin A can be contained at a low dose, for example, in an amount of 5 to 100 ng/mL, preferably 5 to 50 ng/mL, more preferably 5 to 10 ng/mL, in the medium.

In a further alternative embodiment, the concentration of the activin A in the medium is about 0.1 to 100 ng/mL, preferably about 1 to 50 ng/mL, more preferably about 3 to 10 ng/mL.

The concentration of the GSK3β inhibitor in the medium is appropriately set depending on the type of the GSK3β inhibitor used. For example, in the case of using CHIR99021 as the GSK3β inhibitor, its concentration is usually 2 to 5 µM, preferably 2 to 4 µM, particularly preferably about 3 µM.

The concentration of the ROCK inhibitor in the medium is appropriately set depending on the type of the ROCK inhibitor used. For example, in the case of using Y27632 as the ROCK inhibitor, its concentration is usually 5 to 20 µM, preferably 5 to 15 µM, particularly preferably about 10 µM.

The medium can be further supplemented with insulin. The insulin can be contained in an amount of 0.01 to 20 µM, preferably 0.1 to 10 µM, more preferably 0.5 to 5 µM, in the medium. The concentration of the insulin in the medium may be, but is not limited to, the concentration of insulin contained in added B-27 supplement.

In a particular embodiment, the cells are cultured for 1 day in a medium containing activin A, a ROCK inhibitor, and a GSK3β inhibitor and then further cultured for 2 days in a medium containing only activin A with the medium replaced with a fresh one every day. Alternatively, the pluripotent stem cells can be subjected to first culture in a medium containing 0.01 to 20 µM insulin in the presence of a low dose of activin A and subsequently subjected to second culture in an insulin-free medium, for production.

### Step 2) Differentiation into primitive gut tube cells

The definitive endoderm cells obtained in step 1) are further cultured in a medium containing a growth factor to induce their differentiation into primitive gut tube cells. The culture period is 2 days to 8 days, preferably about 4 days.

The culture temperature is not particularly limited, and the culture is performed at 30 to 40°C (for example, 37°C). The concentration of carbon dioxide in a culture container is on the order of, for example, 5%. The culture may be performed by any of two-dimensional culture and three-dimensional culture.

A basal medium for use in the culture of mammalian cells can be used as culture medium, as in step 1). The medium may be appropriately supplemented with a serum replacement, a vitamin, an antibiotic, and the like, in addition to the growth factor.

The growth factor is preferably EGF, KGF, and/or FGF10, more preferably EGF and/or KGF, further preferably KGF.

The concentration of the growth factor in the medium is appropriately set depending on the type of the growth factor used and is usually about 0.1 nM to 1000 µM, preferably about 0.1 nM to 100 µM. In the case of EGF, its concentration is about 5 to 2000 ng/ml (that is, about 0.8 to 320 nM), preferably about 5 to 1000 ng/ml (that is, about 0.8 to 160 nM), more preferably about 10 to 1000 ng/ml (that is, about 1.6 to 160 nM). In the case of FGF10, its concentration is about 5 to 2000 ng/ml (that is, about 0.3 to 116 nM), preferably about 10 to 1000 ng/ml (that is, about 0.6 to 58 nM). For example, in the case of using KGF as the growth factor, its concentration is usually 5 to 150 ng/mL, preferably 30 to 100 ng/mL, particularly preferably about 50 ng/mL.

### Step 3) Differentiation into posterior foregut cells

The primitive gut tube cells obtained in step 2) are further cultured in a medium containing a growth factor, cyclopamine, noggin, and the like to induce their differentiation into posterior foregut cells. The culture period is 1 day to 5 days, preferably about 2 days. The culture may be performed by any of two-dimensional culture and three-dimensional culture.

The culture temperature is not particularly limited, and the culture is performed at 30 to 40°C (for example, 37°C). The concentration of carbon dioxide in a culture container is on the order of, for example, 5%.

As in step 1), a basal medium for use in the culture of mammalian cells can be used as culture medium. The medium may be appropriately supplemented with a serum replacement, a vitamin, an antibiotic, and the like, in addition to the growth factor.

The growth factor is preferably EGF, KGF, and/or FGF10, more preferably EGF and/or KGF, further preferably KGF.

The concentration of the growth factor in the medium is appropriately set depending on the type of the growth factor used and is usually about 0.1 nM to 1000 µM, preferably about 0.1 nM to 100 µM. In the case of EGF, its concentration is about 5 to 2000 ng/ml (that is, about 0.8 to 320 nM), preferably about 5 to 1000 ng/ml (that is, about 0.8 to 160 nM), more preferably about 10 to 1000 ng/ml (that is, about 1.6 to 160 nM). In the case of FGF10, its concentration is about 5 to 2000 ng/ml (that is, about 0.3 to 116 nM), preferably about 10 to 1000 ng/ml (that is, about 0.6 to 58 nM). For example, in the case of using KGF as the growth factor, its concentration is usually 5 to 150 ng/mL, preferably 30 to 100 ng/mL, particularly preferably about 50 ng/mL.

The concentration of the cyclopamine in the medium is not particularly limited and is usually 0.5 to 1.5 µM, preferably 0.3 to 1.0 µM, particularly preferably about 0.5 µM.

The concentration of the noggin in the medium is not particularly limited and is usually 10 to 200 ng/mL, preferably 50 to 150 ng/mL, particularly preferably about 100 ng/mL.

### Step 4) Differentiation into pancreatic progenitor cells

The posterior foregut cells obtained in step 3) are further cultured in a medium containing a factor having CDK8/19-inhibiting activity, preferably a medium containing a factor having CDK8/19-inhibiting activity and a growth factor, to induce their differentiation into pancreatic progenitor cells. The culture period is 2 days to 10 days, preferably about 5 days. The culture may be performed by any of two-dimensional culture and three-dimensional culture.

In the case of two-dimensional culture, according to the previous report (Toyoda et al., Stem cell Research (2015) 14, 185-197), the posterior foregut cells obtained in step 3) are treated with 0.25% trypsin-EDTA solution and dispersed in the solution by pipetting to obtain a cell dispersion, and the obtained dispersion is subjected to centrifugal separation. Recovered cells are resuspended in a small amount of a fresh medium and the cell suspension is reseeded to a fresh medium of step 4).

As in step 1), a basal medium for use in the culture of mammalian cells can be used as culture medium. The medium may be appropriately supplemented with a serum replacement, a vitamin, an antibiotic, and the like, in addition to the growth factor.

Each of the compounds mentioned above or salts thereof can be used as the factor having CDK8/19-inhibiting activity. The amount of the factor added to the medium is appropriately determined according to the compound or the salt thereof used and is usually about 0.00001 µM to 5 µM, preferably 0.00001 µM to 1 µM. The concentration of the factor having CDK8/19-inhibiting activity in the medium is preferably a concentration that attains inhibitory activity of 50% or more for CDK8/19.

The growth factor is preferably EGF, KGF, and/or FGF10, more preferably KGF and/or EGF, further preferably KGF and EGF.

The concentration of the growth factor in the medium is appropriately set depending on the type of the growth factor used and is usually about 0.1 nM to 1000 µM, preferably about 0.1 nM to 100 µM. In the case of EGF, its concentration is about 5 to 2000 ng/ml (that is, about 0.8 to 320 nM), preferably about 5 to 1000 ng/ml (that is, about 0.8 to 160 nM), more preferably about 10 to 1000 ng/ml (that is, about 1.6 to 160 nM). In the case of FGF10, its concentration is about 5 to 2000 ng/ml (that is, about 0.3 to 116 nM), preferably about 10 to 1000 ng/ml (that is, about 0.6 to 58 nM). For example, in the case of using KGF and EGF as the growth factor, the concentration of EGF is usually 5 to 150 ng/mL, preferably 30 to 100 ng/mL, particularly preferably about 50 ng/mL, and the concentration of KGF is usually 10 to 200 ng/mL, preferably 50 to 150 ng/mL, particularly preferably about 100 ng/mL.

Culture on the first day in step 4) may be performed in the presence of a ROCK inhibitor, and culture on the following days may be performed in a medium containing no ROCK inhibitor.

The medium may also contain a protein kinase C (PKC) activator. PdBU (PKC activator II), TPB (PKC activator V), or the like is used as the PKC activator, though the PKC activator is not limited thereto. The concentration of the PKC activator to be added is about 0.1 to 100 ng/ml, preferably about 1 to 50 ng/ml, more preferably about 3 to 10 ng/ml.

The medium may also be supplemented with dimethyl sulfoxide and/or activin (1 to 50 ng/ml).

In any of the steps, the medium may be supplemented with a serum replacement (for example, B-27 supplement, ITS-G), in addition to the components described above. Also, an amino acid, L-glutamine, GlutaMAX (product name), a non-essential amino acid, a vitamin, nicotinamide, an antibiotic (for example, Antibiotic-Antimycotic, penicillin, streptomycin, or a mixture thereof), an antimicrobial agent (for example, amphotericin B), an antioxidant, pyruvic acid, a buffer, inorganic salts, and the like may be added thereto, if necessary. In the case of adding an antibiotic to the medium, its concentration in the medium is usually 0.01 to 20% by weight, preferably 0.1 to 10% by weight. The culture may be performed by any of two-dimensional culture and three-dimensional culture.

In the case of two-dimensional culture, the cell culture is performed by adherent culture without the use of feeder cells. For the culture, a culture container, for example, a dish, a flask, a microplate, or a cell culture sheet such as OptiCell (product name) (Nunc), is used. The culture container is preferably surface-treated in order to improve adhesiveness to cells (hydrophilicity), or coated with a substrate for cell adhesion such as collagen, gelatin, poly-L-lysine, poly-D-lysine, laminin, fibronectin, Matrigel (for example, BD Matrigel (Nippon Becton Dickinson Company, Ltd.)), or vitronectin. The culture container is preferably a culture container coated with type I-collagen, Matrigel, fibronectin, vitronectin or poly-D-lysine, more preferably a culture container coated with Matrigel or poly-D-lysine.

The culture temperature is not particularly limited, and the culture is performed at 30 to 40°C (for example, 37°C). The concentration of carbon dioxide in a culture container is on the order of, for example, 5%.

The pancreatic progenitor cells obtained in step 4) can be further purified using a known surface marker glycoprotein 2 (GP2) or the like. The purification can be performed by a method known per se, for example, using anti-GP2 antibody-immobilized beads.

### Step 5) Differentiation into endocrine progenitor cells

The pancreatic progenitor cells obtained in step 4) are further cultured in a medium containing a growth factor to induce their differentiation into endocrine progenitor cells. The culture may be performed by any of two-dimensional culture and three-dimensional culture. In the case of two-dimensional culture, the pancreatic progenitor cells obtained in step 4) are treated with 0.25% trypsin-EDTA solution and dispersed in the solution by pipetting to obtain a cell dispersion, and the obtained dispersion is subjected to centrifugal separation. Recovered cells are resuspended in a small amount of a fresh medium and the cell suspension is reseeded to a fresh medium of step 5). The culture period is 2 days to 3 days, preferably about 2 days.

As in step 1), a basal medium for use in the culture of mammalian cells can be used as culture medium. The medium is supplemented with SANT1, retinoic acid, ALK5 inhibitor II, T3, and LDN according to the previous report (Nature Biotechnology 2014; 32: 1121-1133) and may be appropriately further supplemented with a Wnt inhibitor, a ROCK inhibitor, FGF (preferably FGF2), a serum replacement, a vitamin, an antibiotic, and the like.

The culture is performed by nonadherent culture without the use of feeder cells. For the culture, a dish, a flask, a microplate, a porous plate (Nunc), or the like, or a bioreactor is used. The culture container is preferably surface-treated in order to decrease adhesiveness to cells.

The culture temperature is not particularly limited, and the culture is performed at 30 to 40°C (for example, 37°C). The concentration of carbon dioxide in a culture container is on the order of, for example, 5%.

The endocrine progenitor cells obtained in step 5) can be further purified using a known surface marker glycoprotein 2 (GP2) or the like. The purification can be performed by a method known per se, for example, using anti-GP2 antibody-immobilized beads.

### Step 6) Differentiation into iPIC

The endocrine progenitor cells obtained in step 5) are further cultured in a medium containing a growth factor to induce their differentiation into iPIC. The culture period is 10 days to 30 days, preferably about 10 to 20 days.

As in step 1), a basal medium for use in the culture of mammalian cells can be used as culture medium. The medium is supplemented with ALK5 inhibitor II, T3, LDN, γ-secretase inhibitor XX, γ-secretase inhibitor RO, N-cysteine, an AXL inhibitor, and ascorbic acid according to the previous report (Nature Biotechnology 2014; 32: 1121-1133) and may be appropriately further supplemented with a Wnt inhibitor, a ROCK inhibitor, FGF (preferably FGF2), a serum replacement, a vitamin, an antibiotic, and the like. For example, the medium may be supplemented with ALK5 inhibitor II, T3, LDN, γ-secretase inhibitor RO, and ascorbic acid or may be supplemented with T3, ALK5 inhibitor II, ZnSO₄, heparin, N-acetylcysteine, Trolox, and R428.

The culture may be performed by any of two-dimensional culture and three-dimensional culture. The culture does not employ feeder cells. Three-dimensional culture is performed by nonadherent culture. For the culture, a dish, a flask, a microplate, a porous plate (Nunc), or the like, or a bioreactor is used. The culture container is preferably surface-treated in order to decrease adhesiveness to cells.

The culture temperature is not particularly limited, and the culture is performed at 30 to 40°C (for example, 37°C). The concentration of carbon dioxide in a culture container is on the order of, for example, 5%.

A feature of the fibrin gel sheet of the present invention is to have a larger number of cells (the number of cells with a higher density) that cannot be achieved by a conventional fibrin gel sheet for cell transplantation. The fibrin gel sheet comprises the cells in an amount of more than 1.5 × 10⁶ cells, preferably 2 × 10⁶ or more cells, more preferably 2.5 × 10⁶ or more cells, further preferably 3 × 10⁶ or more cells, still further preferably 4.5 × 10⁶ or more cells, especially preferably 5 × 10⁶ or more cells, per cm². The upper limit thereof is not particularly limited and can be, for example, 10 × 10⁶ or less cells, preferably 7 × 10⁶ or less cells. The range of the number of cells to be embedded in the fibrin gel sheet of the present invention can be expressed using two numeric values respectively selected from the numeric values of the lower limit and the upper limit. The fibrin gel sheet of the present invention can comprise the cells in an amount appropriately selected from the range of, for example, more than 1.5 × 10⁶ cells to 10 × 10⁶ or less cells/cm², preferably 2 × 10⁶ or more cells to 10 × 10⁶ or less cells/cm², more preferably 2.5 × 10⁶ or more cells to 10 × 10⁶ or less cells/cm², further preferably 3 × 10⁶ or more cells to 10 × 10⁶ or less cells/cm², still further preferably 4.5 × 10⁶ or more cells to 10 × 10⁶ or less cells/cm², especially preferably 5 × 10⁶ or more cells to 10 × 10⁶ or less cells/cm².

The iPIC mentioned above usually exists in the state of a cell mass having a size on the order of 100 µm to 200 µm. The fibrin gel sheet of the present invention comprises the iPIC in an amount of more than 3000 masses, preferably 4000 or more masses, more preferably 5000 or more masses, further preferably 6000 or more masses, still further preferably 9000 or more masses, especially preferably 10000 or more masses, in terms of the number of cell masses per cm². The upper limit thereof is not particularly limited and can be, for example, 20000 or less masses, preferably 14000 or less masses. The range of the number of iPIC (cell masses) to be embedded in the fibrin gel sheet of the present invention can be expressed using two numeric values respectively selected from the numeric values of the lower limit and the upper limit. The fibrin gel sheet of the present invention can comprise the iPIC (cell masses) in an amount appropriately selected from the range of, for example, more than 3000 masses to 20000 or less masses/cm², preferably 4000 or more masses to 20000 or less masses/cm², more preferably 5000 or more masses to 20000 or less masses/cm², further preferably 6000 or more masses to 20000 or less masses/cm², still further preferably 9000 or more masses to 20000 or less masses/cm², especially preferably 10000 or more masses to 20000 or less masses/cm².

In the fibrin gel sheet of the present invention, the fibrin gel is formed by allowing thrombin to act on fibrinogen in an appropriate solvent (for example, water or physiological saline). The amounts of the fibrinogen and the thrombin used in the formation of the fibrin gel and a method for mixing them can be appropriately determined on the basis of the method for producing the fibrin gel sheet of the present invention, which will be described below in detail.

In one aspect, a biodegradable material having a particulate form (hereinafter, also referred to as "biodegradable particles") is further embedded in the fibrin gel sheet of the present invention.

In the present invention, "biodegradable particles" are added and suspended, together with the cells, in a fibrinogen solution in the method for producing the fibrin gel sheet of the present invention, which will be described below in detail. The biodegradable particles suppress the precipitation of the cells in the solution and maintain their dispersion and thereby enable the cells to be dispersed and embedded in the finally obtained fibrin gel sheet.

The biodegradable material constituting the "biodegradable particles" can be any substance degradable by hydrolysis or the like *in vivo* and is not particularly limited. Examples thereof include polylactic acid (PLA), polycaprolactone (PCL), polyurethane (PU), polyethylene glycol (PEG), polyhydroxyethyl methacrylate, polyester, polyglycolic acid (PGA), poly(lactic-co-glycolic acid) (PLGA), poly(3-hydroxybutyrate-co-hydroxyvalerate) (PHBV), poly(ethylene-co-vinyl acetate) (PEVA)polyacrylamide, polyethylene oxide, polyethyleneamine, polyhydroxybutyric acid, poly(N-vinylpyrrolidone), polyvinyl alcohol, polypropylene fumarate, polyacrylic acid, poly-e-caprolactone, polymethacrylic acid, polyvinylidene difluoride (PVDF), pectic acid, hyaluronic acid, heparin sulfate, chondroitin sulfate, heparan sulfate proteoglycan, heparin, chitin, chitosan, xanthan, carboxymethylcellulose, carboxymethyl chitosan, alginate, alginic acid ester, collagen, cellulose, silk fibroin, keratin, gelatin, fibrin, pullulan, laminin, gellan, silicon, urethane, elastin and modified forms thereof, and combinations thereof. Preferably, the biodegradable particles are constituted by a gel-like biodegradable material selected from the group consisting of gelatin, collagen, hyaluronic acid, carboxymethylcellulose, PLA, PGA, PLGA, and the like, and modified forms thereof, and combinations thereof.

The form of the "biodegradable particles" can be a form capable of suppressing the precipitation of the cells in the solution and maintaining their dispersion. The biodegradable particles can have any form such as a block, bead, pellet, sheet, or gel form and can be solid or porous. The form of the "biodegradable particles" is preferably a three-dimensional shape. Examples thereof include, but are not limited to, spheres, polyhedrons such as tetrahedrons and hexahedrons, cylinders, prisms, cones, truncated cones, pyramids, truncated pyramids, toruses, discs, ellipsoids, and deformed three-dimensional forms thereof.

The size of the "biodegradable particles" can be a size that can suppress the precipitation of the cells in the solution and maintain their dispersion and does not impair the formation of the fibrin gel sheet of the present invention. The size can be a size with which the biodegradable particles are capable of passing through an opening size on the order of 100 µm to 1000 µm, preferably 100 µm to 800 µm, more preferably 150 µm to 400 µm, further preferably 200 µm.

In one aspect of the present invention, the biodegradable particles are a gelatin gel and have a shape of a sphere capable of passing through an opening size of 200 µm.

The amount of the biodegradable particles contained in the fibrin gel sheet of the present invention can be an amount that can suppress the precipitation of the cells in the fibrinogen solution and maintain their dispersion and does not impair the formation of the fibrin gel sheet of the present invention. The fibrinogen solution can contain the biodegradable particles in an amount of, for example, 10 to 30 w/v%, preferably 15 to 25 w/v%, more preferably 18 to 22 w/v%, further preferably 20 w/v%.

The biodegradable particles in the fibrin gel sheet of the present invention may maintain their form and shape or may be partially or wholly integrated with the fibrin gel.

In another aspect, the fibrin gel sheet of the present invention further comprises a biodegradable gelator.

In the present invention, "biodegradable gelator" is added and dissolved, together with the cells, in a fibrinogen solution in the method for producing the fibrin gel sheet of the present invention, which will be described below in detail. The biodegradable gelator imparts viscosity to the solution and is capable of suppressing the precipitation of the cells in the solution and maintaining their dispersion and thereby enables the cells to be dispersed and embedded in the finally obtained fibrin gel sheet.

The "biodegradable gelator" can be any substance degradable by hydrolysis or the like *in vivo* while the substance can impart viscosity capable of suppressing the precipitation of the cells in the solution and maintaining their dispersion and does not impair the formation of the fibrin gel sheet of the present invention. Thus, the biodegradable gelator is not particularly limited. For example, the biodegradable material mentioned above can be used (except for fibrin). Preferably, a biodegradable material selected from the group consisting of collagen, hyaluronic acid, heparin sulfate, chondroitin sulfate, heparan sulfate proteoglycan, carboxymethylcellulose, alginic acid, cellulose, silk fibroin, keratin, gelatin, dextran, PEG, and the like, and modified forms thereof, and combinations thereof can be used as the biodegradable gelator.

The amount of the biodegradable gelator contained in the fibrin gel sheet of the present invention can be an amount that can suppress the precipitation of the cells in the fibrinogen solution and maintain their dispersion and does not impair the formation of the fibrin gel sheet of the present invention. The fibrinogen solution can contain the biodegradable gelator in an amount of, for example, 0.2 to 2 w/v%, preferably 0.4 to 1.5 w/v%, more preferably 0.5 to 1 w/v%, further preferably 0.75 w/v%.

The fibrin gel sheet of the present invention can further comprise a support. The support is capable of holding up the fibrin gel sheet of the present invention at the time of production and/or use of the fibrin gel sheet while contributing to improvement in handleability of the fibrin gel sheet. The support is made of, but not limited to, the biodegradable material mentioned above or a nonbiodegradable material, for example, polyester (PEs), polypropylene (PP), polyethylene (PE), polytetrafluoroethylene (PTFE), polyethylene terephthalate (PET), nylon, or silk and has any shape (for example, a sheet, mesh, plate, woven fabric, or nonwoven fabric shape) capable of holding up the fibrin gel sheet of the present invention. The support may be allowed to adhere to the surface of the fibrin gel sheet of the present invention or may be partially or wholly fixed in the fibrin gel sheet of the present invention.

In one aspect of the present invention, the fibrin gel sheet of the present invention comprises a support having a mesh-like shape made of polyester or poly(lactic-co-glycolic acid).

The fibrin gel sheet of the present invention may be provided in the form of a laminate in which a plurality of fibrin gel sheets of the present invention are laminated. The laminate may have the same cells embedded in the individual fibrin gel sheets or may be a combination of the fibrin gel sheets in which some or all of the embedded cells are different.

### 3. Method for producing fibrin gel sheet of present invention

The present invention also relates to a method for producing the fibrin gel sheet of the present invention (hereinafter, also referred to as "method of the present invention"). The method comprises the step of allowing thrombin to act on a fibrinogen solution of the cells suspended therein, and shaping a sheet that has a size of 2.25 cm² or larger in terms of a surface area of one surface and has a thickness of 1 mm or smaller, followed by gelation (solidification). The fibrin gel can be obtained by mixing fibrinogen and thrombin in an appropriate solvent (for example, water or physiological saline), followed by gelation (solidification).

The cells are uniformly dispersed and suspended in the fibrinogen solution. The amount of the cells can be determined on the basis of the fibrin gel sheet after shaping. Specifically, the amount is more than 1.5 × 10⁶ cells, preferably 2 × 10⁶ or more cells, more preferably 2.5 × 10⁶ or more cells, further preferably 3 × 10⁶ or more cells, still further preferably 4.5 × 10⁶ or more cells, especially preferably 5 × 10⁶ or more cells, per cm² of the fibrin gel sheet. The upper limit thereof is not particularly limited, and the cells can be suspended in an amount of, for example, 10 × 10⁶ or less cells, preferably 7 × 10⁶ or less cells. The range of the number of cells to be suspended in the fibrinogen solution can be expressed using two numeric values respectively selected from the numeric values of the lower limit and the upper limit. The cells can be suspended in the fibrinogen solution in an amount appropriately selected from the range of, for example, more than 1.5 × 10⁶ cells to 10 × 10⁶ or less cells/cm², preferably 2 × 10⁶ or more cells to 10 × 10⁶ or less cells/cm², more preferably 2.5 × 10⁶ or more cells to 10 × 10⁶ or less cells/cm², further preferably 3 × 10⁶ or more cells to 10 × 10⁶ or less cells/cm², still further preferably 4.5 × 10⁶ or more cells to 10 × 10⁶ or less cells/cm², especially preferably 5 × 10⁶ or more cells to 10 × 10⁶ or less cells/cm², in the fibrin gel sheet.

In one embodiment of the method of the present invention, when the cells are iPIC, the iPIC in the fibrinogen solution is in an amount of more than 3000 masses, preferably 4000 or more masses, more preferably 5000 or more masses, further preferably 6000 or more masses, still further preferably 9000 or more masses, especially preferably 10000 or more masses, in terms of the number of cell masses per cm² of the fibrin gel sheet. The upper limit thereof is not particularly limited and, the cells can be suspended in an amount of, for example, 20000 or less masses, preferably 14000 or less masses. The range of the number of iPIC (cell masses) to be suspended in the fibrinogen solution can be expressed using two numeric values respectively selected from the numeric values of the lower limit and the upper limit. The iPIC (cell masses) can be suspended in an amount appropriately selected from the range of, for example, more than 3000 masses to 20000 or less masses/cm², preferably 4000 or more masses to 20000 or less masses/cm², more preferably 5000 or more masses to 20000 or less masses/cm², further preferably 6000 or more masses to 20000 or less masses/cm², still further preferably 9000 or more masses to 20000 or less masses/cm², especially preferably 10000 or more masses to 20000 or less masses/cm², in the fibrin gel sheet.

In the shaping of the fibrin gel sheet, the surface area of one surface (that is, any of the major surfaces of the sheet or a surface on one side of the sheet) of the sheet is 2.25 cm² or larger, preferably 4 cm² or larger, more preferably 9 cm² or larger, further preferably 16 cm² or larger, still further preferably 25 cm² or larger, especially preferably 36 cm² or larger, especially more preferably 49 cm² or larger, especially further preferably 64 cm² or larger, especially still further preferably 81 cm² or larger, particularly preferably 100 cm² or larger. The upper limit thereof is not particularly limited and may be appropriately selected depending on the size or shape of a transplantation site. The size can be, for example, 400 cm² or smaller.

The range of the surface area in the fibrin gel sheet obtained by shaping can be expressed using two numeric values respectively selected from the numeric values of the lower limit and the upper limit. The surface area of the fibrin gel sheet obtained by shaping can be a size appropriately selected from the range of, for example, 2.25 cm² or larger to 400 cm² or smaller, preferably 4 cm² or larger to 400 cm² or smaller, more preferably 9 cm² or larger to 400 cm² or smaller, further preferably 16 cm² or larger to 400 cm² or smaller, still further preferably 25 cm² or larger to 400 cm² or smaller, especially preferably 36 cm² or larger to 400 cm² or smaller, especially more preferably 49 cm² or larger to 400 cm² or smaller, especially further preferably 64 cm² or larger to 400 cm² or smaller, especially still further preferably 81 cm² or larger to 400 cm² or smaller, particularly preferably 100 cm² or larger to 400 cm² or smaller.

The shape of the one surface of the fibrin gel sheet obtained by shaping is not particularly limited and may be appropriately selected depending on the size or shape of a transplantation site. Examples thereof include, but are not limited to, polygons (for example, triangles, tetragons, pentagons, hexagons, and octagons), rounded polygons, round shapes, and oval shapes.

When the fibrin gel sheet obtained by shaping has, for example, a tetragon, the length is 15 mm or larger, preferably 20 mm or larger, more preferably 30 mm or larger, further preferably 40 mm or larger, still further preferably 50 mm or larger, especially preferably 60 mm or larger, especially more preferably 70 mm or larger, especially further preferably 80 mm or larger, especially still further preferably 90 mm or larger, particularly preferably 100 mm or larger. The upper limit thereof is not particularly limited and can be, for example, 200 mm or smaller. The width is 15 mm or larger, preferably 20 mm or larger, more preferably 30 mm or larger, further preferably 40 mm or larger, still further preferably 50 mm or larger, especially preferably 60 mm or larger, especially more preferably 70 mm or larger, especially further preferably 80 mm or larger, especially still further preferably 90 mm or larger, particularly preferably 100 mm or larger. The upper limit thereof is not particularly limited and can be, for example, 200 mm or smaller.

The range of the length and the width can be expressed using two numeric values respectively selected from the numeric values of the lower limit and the upper limit. The size (length × width (which may be width × length)) of the fibrin gel sheet obtained by shaping can be a size appropriately selected from the range of, for example, 15 mm or larger to 200 mm or smaller × 15 mm or larger to 200 mm or smaller, preferably 20 mm or larger to 200 mm or smaller × 20 mm or larger to 200 mm or smaller, more preferably 30 mm or larger to 200 mm or smaller × 30 mm or larger to 200 mm or smaller, further preferably 40 mm or larger to 200 mm or smaller × 40 mm or larger to 200 mm or smaller, still further preferably 50 mm or larger to 200 mm or smaller × 50 mm or larger to 200 mm or smaller, especially preferably 60 mm or larger to 200 mm or smaller × 60 mm or larger to 200 mm or smaller, especially more preferably 70 mm or larger to 200 mm or smaller × 70 mm or larger to 200 mm or smaller, especially further preferably 80 mm or larger to 200 mm or smaller × 80 mm or larger to 200 mm or smaller, especially still further preferably 90 mm or larger to 200 mm or smaller × 90 mm or larger to 200 mm or smaller, particularly preferably 100 mm or larger to 200 mm or smaller × 100 mm or larger to 200 mm or smaller. Examples of the size (length × width (which may be width × length)) of the fibrin gel sheet obtained by shaping include, but are not limited to, 15 mm or larger × 15 mm or larger, preferably 20 mm × 20 mm, 20 mm × 30 mm, or 30 mm × 30 mm, more preferably 40 mm × 40 mm, 40 mm × 50 mm, 50 mm × 50 mm, 40 mm × 60 mm, or 60 mm × 60 mm, further preferably 70 mm × 70 mm, 80 mm × 80 mm, 90 mm × 90 mm, 100 mm × 100 mm, or 200 mm × 200 mm.

The thickness of the fibrin gel sheet obtained by shaping can be appropriately set depending on a factor such as the size or amount of the cells to be embedded or the size of a transplantation site and is preferably 1 mm or smaller. When the thickness of the fibrin gel sheet obtained by shaping is 1 mm or smaller, the supply of a nutrient or oxygen to the cells embedded in the fibrin gel sheet, for example, can be efficiently performed and rapid degradation of the fibrin gel after transplantation can be achieved, which are preferred. The thickness of the fibrin gel sheet obtained by shaping can be, for example, 1 mm or smaller, 900 µm or smaller, 800 µm or smaller, 700 µm or smaller, 600 µm or smaller, 500 µm or smaller, or 400 µm or smaller. The lower limit thereof is not particularly limited and can be, for example, 100 µm or larger, 200 µm or larger, or 300 µm or larger. The range of the thickness of the fibrin gel sheet obtained by shaping can be expressed using two numeric values respectively selected from the numeric values of the upper limit and the lower limit. The thickness of the fibrin gel sheet obtained by shaping can be, for example, 100 µm or larger to 1 mm or smaller, 100 µm to 800 µm or smaller, 200 µm to 600 µm or smaller, or 300 µm or larger to 400 µm or smaller and can be a thickness appropriately selected from these ranges. The thickness of the fibrin gel sheet obtained by shaping can be, for example, about 400 µm. The thickness of the fibrin gel sheet obtained by shaping does not have to be uniform throughout the sheet, and the thickest part can fall within the range described above.

In one aspect of the method of the present invention, the amounts of fibrinogen and thrombin blended and/or a temperature at the time of shaping is adjusted, whereby the gelation (solidification) rate of the fibrin gel to be formed can be adjusted, and the fibrin gel can be expanded to shape a fibrin gel sheet having a desired size.

In this aspect, the fibrinogen solution contains the fibrinogen in an amount of 5 to 150 mg/mL, preferably 10 to 80 mg/mL, more preferably 20 mg/mL, and the thrombin in an amount of 0.4 U or less, preferably 0.3 U or less or 0.25 U or less, more preferably 0.2 U or less or 0.15 U or less, further preferably 0.1 U or less or 0.08 U or less with respect to 1 mg of the fibrinogen. The lower limit thereof is not particularly limited, and the thrombin is allowed to act in an amount that attains a blending ratio of 0.04 U or more. If the amounts of the fibrinogen and/or the thrombin blended are larger than the above range, gelation (solidification) may progress rapidly and make it difficult or impossible to expand the gel and shape a sheet having a desired size. On the other hand, if the amounts of the fibrinogen and/or the thrombin blended are smaller than the above range, gelation (solidification) may be insufficient and make it difficult or impossible to shape a sheet having a desired size. The thrombin can be allowed to act in any form on the fibrinogen and may be added directly to the fibrinogen solution or may be dissolved in an appropriate solvent (for example, water or physiological saline) to prepare a thrombin solution in advance, which can be mixed with the fibrinogen solution.

Any approach can be used as an approach of shaping a sheet having a desired size and thickness from the fibrinogen solution on which the thrombin has been allowed to act. For example, any molding approach conventionally known in the field of polymer sheet production (for example, cast molding, extrusion molding, calendar molding, inflation molding) can be performed singly or in combination. Preferably, cast molding is performed. In this aspect, the fibrinogen solution on which the thrombin has been allowed to act may be spread out to shape a sheet having a desired size and thickness, and cast molding, that is, shaping by injecting the fibrinogen solution to a sheet mold having the desired size and thickness and spreading out the solution using a spatula, a pallet, or the like, if necessary, can be easily carried out.

The sheet is shaped under cooling from the fibrinogen solution on which the thrombin has been allowed to act. The fibrin gel solution is placed under cooling and can thereby shape a sheet having a desired size and thickness, more specifically, can thereby be expanded to shape such a sheet, while a gelation (solidification) rate is suppressed. The cooling temperature can be a temperature that enables the fibrin gel solution to shape a sheet having a desired size and thickness, and is not particularly limited. The temperature is, for example, 2 to 8°C, preferably 2 to 6°C, more preferably 4°C. The cooling is not limited as long as the temperature of the fibrin gel solution is lowered. The fibrin gel solution may be cooled, or a shaping unit (for example, a mold) in contact with the fibrin gel solution may be cooled.

Before gelation (solidification) of the shaped sheet of the fibrin gel solution, the support mentioned above may be integrated with the fibrin gel sheet, if necessary. The support may be allowed to adhere to the surface of the fibrin gel solution or may be partially or wholly dipped in the fibrin gel solution. The resultant can be allowed to gelate (solidified) to obtain a fibrin gel sheet integrally comprising the support.

In another aspect of the method of the present invention, "biodegradable particles" are added and suspended in a fibrinogen solution of the cells suspended therein, or "biodegradable gelator" is added and dissolved therein, whereby the cells are prevented from being precipitated in the fibrinogen solution so that a uniformly dispersed and suspended state of the cells can be maintained in the fibrinogen solution to produce a fibrin gel sheet in which the cells are dispersed and embedded.

"Biodegradable particles" defined above can be used and can be added and suspended in an amount that can suppress the precipitation of the cells in the fibrinogen solution of the cells suspended therein and maintain their dispersion and does not impair the formation of the fibrin gel sheet of the present invention. The biodegradable particles are added and suspended in an amount of, for example, 10 to 30 w/v%, preferably 15 to 25 w/v%, more preferably 18 to 22 w/v%, further preferably 20 w/v%, in the fibrinogen solution of the cells suspended therein. In one embodiment of this aspect, the biodegradable particles are gelatin gel particles that have a spherical shape capable of passing through an opening size of 200 µm and are added and suspended in an amount of 20 w/v% in the fibrinogen solution of the cells suspended therein.

"Biodegradable gelator" defined above can be used and can be added and dissolved in an amount that can suppress the precipitation of the cells in the fibrinogen solution of the cells suspended therein and maintain their dispersion and does not impair the formation of the fibrin gel sheet of the present invention. The biodegradable gelator is added and dissolved in an amount of, for example, 0.2 to 2 w/v%, preferably 0.4 to 1.5 w/v%, more preferably 0.5 to 1 w/v%, further preferably 0.75 w/v%, in the fibrinogen solution of the cells suspended therein. In one embodiment of this aspect, the biodegradable gelator is collagen that is added and dissolved in an amount of 0.75 w/v% in the fibrinogen solution of the cells suspended therein.

In this aspect, the fibrinogen solution contains the fibrinogen in an amount of 5 to 150 mg/mL, preferably 20 to 100 mg/mL, more preferably 80 mg/mL. The thrombin can be allowed to act in an amount sufficient for the gelation (solidification) of the fibrinogen solution and is allowed to act in an amount of, for example, 0.5 to 5 U, preferably 1 to 4 U, more preferably 1.5 to 3.5 U, with respect to 1 mg of the fibrinogen. The thrombin can be allowed to act in any form on the fibrinogen and can be allowed to act by adding the thrombin directly to the fibrinogen solution, by dissolving the thrombin in an appropriate solvent (for example, water or physiological saline) to prepare a thrombin solution in advance, which is then added to the fibrinogen solution, by allowing the thrombin to be contained in "base material" described below in detail, or by combining two or more of these approaches. When the base material contains the thrombin, the fibrinogen and the thrombin can be mixed by applying the fibrinogen solution to the base material.

Any approach can be used as an approach of shaping a sheet having a desired size and thickness from the fibrinogen solution of the cells suspended therein, for example, by applying the fibrin gel solution to an appropriate base material, or by using any molding approach conventionally known in the field of polymer sheet production (for example, cast molding, extrusion molding, calendar molding, inflation molding), or a combination of one or more of these methods, preferably by applying the fibrin gel solution to a suitable substrate. This aspect enables uniform dispersion of suspended cells to be maintained, and a sheet in which cells are uniformly dispersed can be easily shaped not only when the fibrinogen solution of the cells suspended therein is injected at once but when this solution is continuously injected (over time from the start to the end of injection).

"Base material" is not particularly limited as long as the base material has a plane surface that permits sheet formation on the surface. Preferably, the support mentioned above can be used as the base material. The fibrinogen solution of the cells suspended therein can be applied to the base material by any approach. The application can be performed manually and/or mechanically using one or more of injection, a spatula, a pallet, a spray, and a bioprinter (2D or 3D printer), for example, and is preferably performed mechanically using a bioprinter.

The fibrin gel sheet of the present invention produced by the method of the present invention can be preserved before use in a medium for cell culture, for example, but not limited to, Dulbecco's modified Eagle medium (DMEM), RPMI1640 medium, MEM medium, or CMRL medium, a buffer solution, for example, but not limited to, phosphate-buffered saline (PBS) or Hank's balanced salt solution (HBSS), or an isotonic solution, for example, but not limited to, physiological saline.

### 4. Application of fibrin gel sheet of present invention

The fibrin gel sheet of the present invention has functions equivalent or similar to cells constituting organs or tissues of, for example, but not limited to, the epidermis, the nerve, the brain, the spinal cord, the esophagus, the stomach, the small intestine, the large intestine, the urinary bladder, the urethra, the lung, the thyroid gland, the pancreas, the liver, muscle, skeleton, the heart, vascular vessel, the spleen, the kidney, and blood, or precursor cells thereof, depending on the dispersed and embedded cells mentioned above, and can be used in a cell therapy method using cells in order to enhance or maintain the functions of these organs or tissues or in order to assist or replenish the functions of these organs or tissues reduced or eliminated due to diseases, disorders, or the like. The fibrin gel sheet of the present invention is used for transplantation to a patient in need thereof.

In one embodiment of the present invention, when the fibrin gel sheet of the present invention comprises pluripotent stem cell-derived iPIC, the fibrin gel sheet of the present invention can improve and/or maintain a blood glucose level in a recipient patient by the action of insulin or glucagon secreted by the iPIC, and can control the blood glucose level to a normal level. Thus, the fibrin gel sheet of the present invention comprising iPIC can be used for treating or preventing a disease, a disorder, or a symptom that requires improving and/or maintaining a blood glucose level. Examples of such a disease, a disorder, or a symptom include, but are not limited to, diabetes mellitus (type 1 diabetes mellitus and type 2 diabetes mellitus), alteration of glucose levels on an empty stomach and after eating, and hypoglycemia (for example, hypoglycemia caused by the administration of insulin to diabetic patients). "Treatment" means treatment, curing, prevention, or amelioration of remission of a disease, a disorder, or a symptom, or reduction of the progression speed of a disease, a disorder, or a symptom. "Prevention" means reduction of the possibility or risk of the onset of a disease, a disorder, or a symptom, or retardation of the onset of a disease, a disorder, or a symptom.

The recipient for the transplantation of the fibrin gel sheet of the present invention is a patient in need thereof for cell therapy. Examples thereof include, but are not limited to, mammals such as mice, rats, hamsters, rabbits, cats, dogs, bovines, sheep, monkeys, and humans. A large mammal is preferred, and a human is particularly preferred.

When the fibrin gel sheet of the present invention is transplanted, the fibrin gel is rapidly degraded so that the embedded cells can be in rapid contact with host tissues and can receive oxygen, nutrients, and the like. Hence, the fibrin gel sheet of the present invention may be transplanted to tissues poor in vascular vessels (for example, subcutaneous tissues), and angiogenesis does not have to be performed beforehand for transplantation and is not essential.

The fibrin gel sheet of the present invention may be transplanted as one or more sheets depending on symptoms of a patient or the size of a transplantation site. The fibrin gel sheet of the present invention may be transplanted in the form of a laminate of a plurality of sheets, if necessary, because of its small thickness.

Hereinafter, the present invention will be described with reference to Examples. However, the present invention is not limited by these Examples.

### Examples

### Production of iPIC

iPIC was prepared by the induction of differentiation from human iPS cells (Ff-I14-s04 line) according to the above steps 1)-6) and the previous reports (Stem Cell Research (2015) 14, 185-197; and Nature Biotechnology 2014; 32: 1121-1133).

Specifically, the pancreatic progenitor cells prepared by the induction of differentiation from human iPS cells was cultured for 2 days in a medium for induction of differentiation (Improved MEM/1% B-27/Penicillin Streptomycin medium) containing differentiation factors (SANT1, retinoic acid, T3, LDN, a Wnt inhibitor, a ROCK inhibitor, and FGF2) as well as ALK5iII (10 µM) and thereby induced to differentiate into an endocrine progenitor cells.

Subsequently, the endocrine progenitor cells were cultured for 7 days in a medium for induction of differentiation (Improved MEM/1% B-27/Penicillin Streptomycin medium) containing differentiation factors (T3, LDN, γ-secretase inhibitor RO, and FGF receptor 1 inhibitor PD-166866) as well as ALK5iII (10 µM) and then cultured for 4 days in a medium for induction of differentiation (MCDB131/20 mM Glucose/NaHCO₃/FAF-BSA/ITS-X/Glutamax/Penicillin Streptomycin medium) containing T3, LDN, γ-secretase inhibitor RO, N-acetylcysteine, AXL inhibitor R428, ascorbic acid, a ROCK inhibitor, ZnSO₄, heparin, and Trolox as well as ALK5iII (10 µM) and thereby induced to differentiate into iPIC.

The obtained iPIC had the form of aggregates (spheroids) (diameter: about 150 µm) each containing about 500 aggregated cells.

### Example 1 Evaluation of transplantation shape

### <Method>

### Preparation of fibrin gel mass

3 × 10⁶ cells of iPIC (about 6000 aggregates) were collected into a 1.5 mL tube and centrifuged. Then, a medium supernatant was removed, and the cells were suspended by the addition of 25 µL of a fibrinogen solution (80 mg/mL). 25 µL of a thrombin solution (125 U/mL) was further added thereto, and the resultant was left standing for 5 minutes for gelation to prepare an iPIC-embedded fibrin gel mass (diameter: about 5 mm).

### Preparation of fibrin gel sheet

3 × 10⁶ cells of iPIC were collected into a 1.5 mL tube and centrifuged. Then, a medium supernatant was removed, and the cells were suspended by the addition of 25 µL of a fibrinogen solution (80 mg/mL). The fibrinogen solution of the iPIC suspended therein was thinly applied onto a polyester mesh (aperture ratio: 69%, 10 × 10 mm) impregnated with 12.5 µL of a thrombin solution (125 U/mL) and then 25 µL of a thrombin solution was wholly applied, and the resultant was left standing for 5 minutes for gelation to prepare an iPIC-embedded thin-layer fibrin gel sheet.

### In vivo evaluation

The iPIC-embedded fibrin gel mass or the iPIC-embedded fibrin gel sheet was subcutaneously transplanted to a nude rat under anesthesia. After transplantation, blood was collected from the tail vein, and plasma was separated, followed by the measurement of a human c-peptide concentration by ELISA.

### <Results>

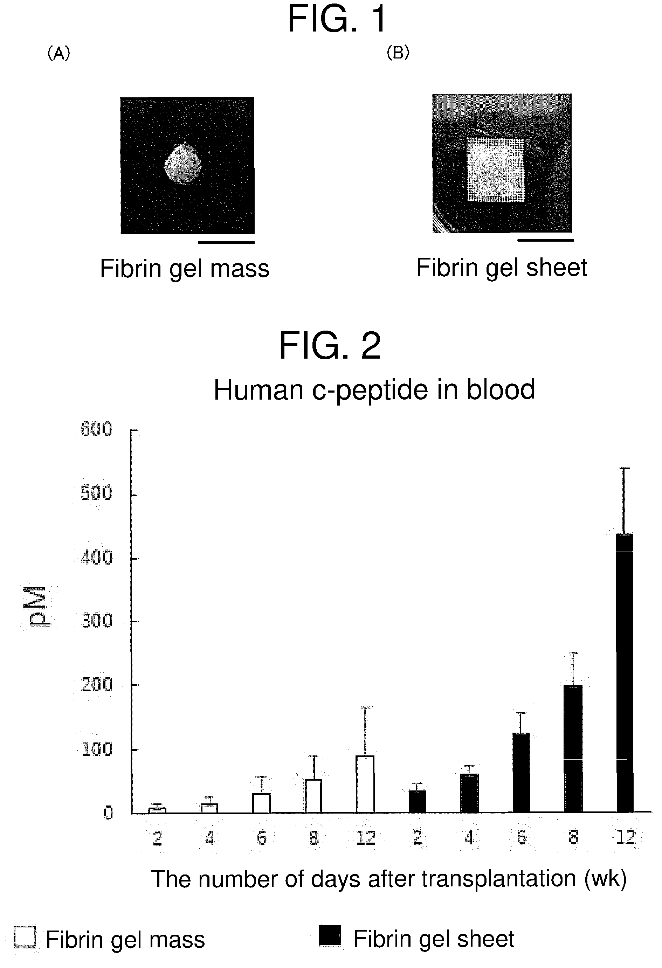

Figure 1 shows the appearance of the fibrin gel mass and the fibrin gel sheet. Both the specimens retained iPIC within the gel and were found to have no leakage of cells.

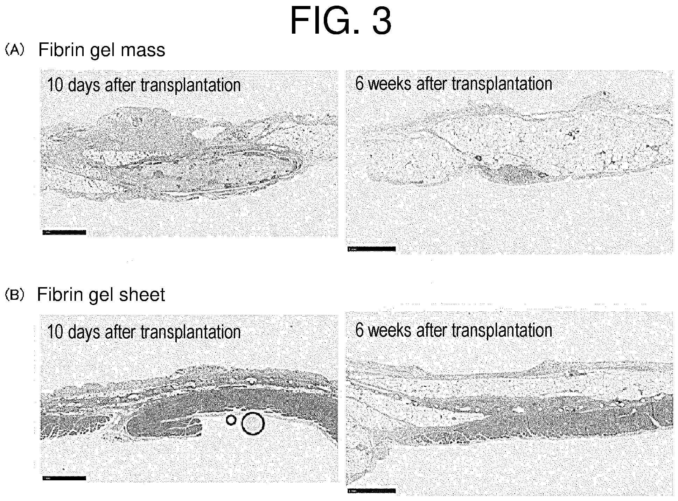

Figure 2 shows results of measuring a human c-peptide concentration in blood over time in the nude rat in which the iPIC-embedded fibrin gel mass or the iPIC-embedded fibrin gel sheet was subcutaneously transplanted. Figure 3 shows histological analysis results about each transplantation site 10 days and 6 weeks after transplantation.

In the results of Figure 2, the human c-peptide concentration in blood exhibited a higher value by the transplantation of the iPIC-embedded fibrin gel in a sheet shape than that in a mass shape.

From the histological analysis results 10 days after transplantation shown in Figure 3, the fibrin gel in the mass was degraded only at its periphery so that iPIC in this portion was in contact with host tissues, while most parts of the fibrin gel remained where iPIC in no contact with host tissues was found. On the other hand, almost the whole fibrin gel in the sheet was rapidly degraded so that the transplanted iPIC was in contact with host tissues.

6 weeks after transplantation, the whole gel even in the transplanted mass was degraded, whereas the human c-peptide concentration in blood exhibited a higher value for the transplanted sheet. These results suggested that even in the case of using a biodegradable material gel, it is important for the survival of transplanted cells to rapidly degrade the gel early after transplantation and supply oxygen, nutrients, and the like to the transplanted cells in contact with a host and this also influences subsequent long-term drug efficacy, indicating the usefulness of transplantation in a thin-layer sheet shape.

### Example 2 Preparation of large animal/human-sized fibrin gel sheet (production method 1_method using cooled metal mold)

### <Method>

### Evaluation of gelation time

A fibrinogen solution (40 mg/mL) and a thrombin solution (31 U/mL, 15.6 U/mL, 10.4 U/mL, 5.2 U/mL, and 3.1 U/mL) were prepared, and 440 µL each of the solutions were combined in a 1.5 mL tube, mixed by pipetting once or twice, and then evaluated for a gelation status according to the following criteria to analyze the relationship between a thrombin concentration and a gelation time.

### Criteria for evaluation of gelation status:

○: The pipetting operation is possible in the tube.
△: Partial gelation occurs, though the pipetting operation is possible.
×: Whole gelation occurs and makes the pipetting operation impossible.

### Preparation of large animal/human-sized fibrin gel sheet

45 × 10⁶ cells of iPIC were collected into a 15 mL tube and centrifuged. Then, a medium supernatant was removed, and the cells were suspended by the addition of 360 µL of a fibrinogen solution (40 mg/mL). Then, the cells were suspended by the addition of 360 µL of a thrombin solution (15.6 U/mL) and then immediately spread on a metal mold (40 × 60 × 0.4 mm) cooled to 4°C in advance. A polyester mesh (aperture ratio: 69%, size: 40 × 10 mm) was further placed thereon, and the resultant was left standing for 6 minutes at room temperature for gelation.

### <Results>

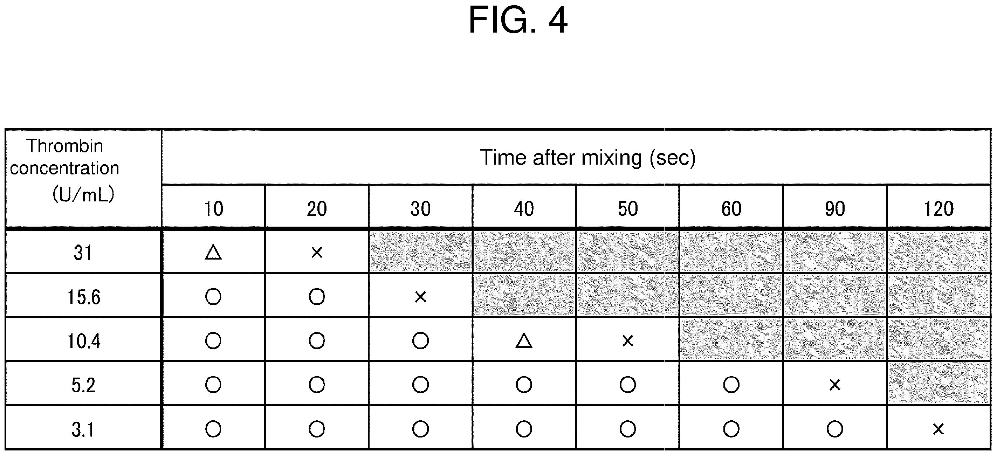

As a result of analyzing the relationship between a thrombin concentration and a gelation time, the gelation time was delayed with decrease in thrombin concentration, and a concentration of 15.6 U/mL or lower was shown to be able to secure a time necessary for shaping a 40 × 60 × 0.4 mm fibrin gel sheet (Figure 4).

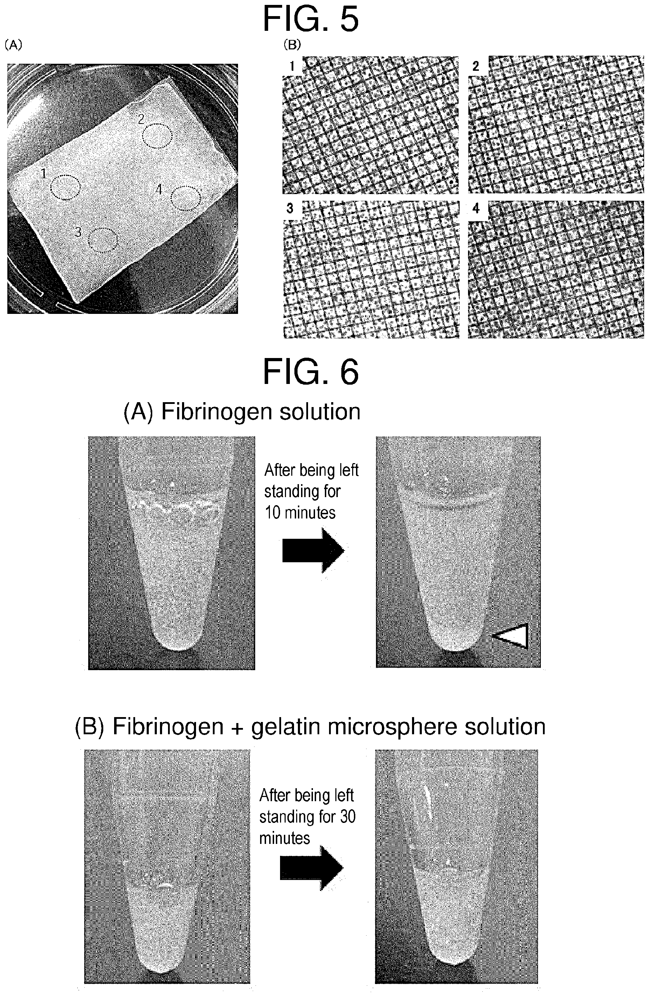

A 40 × 60 × 0.4 mm fibrin gel sheet available for large animals and humans was able to be prepared by rapidly spreading a fibrinogen solution (40 mg/mL) and a thrombin solution (15.6 U/mL) on a cooled metal mold (Figure 5). As a result of further confirming the obtained fibrin gel sheet under a phase-contrast microscope, it was able to be confirmed that the embedded iPIC was uniformly dispersed at any position (in Figure 5(B), iPIC is observed in a black particulate form).

### Example 3 Preparation of large animal/human-sized fibrin gel sheet (production method 2-1_fibrin + gelatin microsphere gel sheet)

### <Method>

### Confirmation of retention of cell dispersibility

Gelatin was dissolved at 2 w/v% using physiological saline and cooled in a refrigerator of 4°C for gelation. Then, the gelatin gel was allowed to pass through a mesh having an opening size of 200 µm to prepare an about 200 µm microsphere of the gelatin gel (hereinafter, referred to as "gelatin microsphere"). The gelatin microsphere was suspended at 20 w/v% in a fibrinogen solution (40 mg/mL) to prepare a fibrinogen + gelatin microsphere solution. iPIC was suspended in a fibrinogen solution (40 mg/mL) or the fibrinogen + gelatin microsphere solution, and the resultant was then left standing and evaluated for the retention of a dispersed state of the cells from the presence or absence of precipitate formation.

### In vivo test

3 × 10⁶ cells of iPIC were collected into a 1.5 mL tube and centrifuged. Then, a medium supernatant was removed, and the cells were suspended by the addition of 25 µL of the fibrinogen + gelatin microsphere solution prepared as described above. The fibrinogen + gelatin microsphere solution of the iPIC suspended therein was thinly applied onto a polyester mesh (aperture ratio: 69%, size: 10 × 10 mm) impregnated with 12.5 µL of a thrombin solution (62.5 U/mL)and then 25 µL of a thrombin solution was wholly applied, and the resultant was left standing for 5 minutes for gelation to prepare an iPIC-embedded thin-layer fibrin + gelatin microsphere gel sheet.

The iPIC-embedded fibrin gel sheet described in Example 1 or the iPIC-embedded fibrin + gelatin microsphere gel sheet was subcutaneously transplanted to a nude rat under anesthesia. After transplantation, blood was collected from the tail vein, and plasma was separated, followed by the measurement of a human c-peptide concentration by ELISA.

### Preparation of large animal/human-sized gel sheet

75 × 10⁶ cells of iPIC were collected into a 15 mL tube and centrifuged. Then, a medium supernatant was removed, and the cells were suspended by the addition of 600 µL of the fibrinogen + gelatin microsphere solution. The fibrinogen + gelatin microsphere solution of the iPIC suspended therein was thinly applied onto a polyester mesh (aperture ratio: 69%, size: 40 × 60 mm) or a glycolic acid/lactate polyester (90/10, poly(glycolideco-L-lactide), size: 40 × 60 mm) mesh impregnated with 300 µL of a thrombin solution (62.5 U/mL) by using a pipette or a 3D printer and then 600 µL of a thrombin solution was wholly applied, and the resultant was left standing for 5 minutes for gelation to prepare an iPIC-embedded thin-layer fibrin + gelatin microsphere gel sheet.

### <Results>

Figure 6 shows the state of iPIC suspended in the fibrinogen solution or the fibrinogen + gelatin microsphere solution and left standing. In the fibrinogen solution (Figure 6(A)), iPIC was found to be precipitated after being left standing for 10 minutes (arrowhead). On the other hand, in the fibrinogen + gelatin microsphere solution (Figure 6(B)), iPIC maintained its dispersed state even after being left standing for 30 minutes.

The produced iPIC-embedded thin-layer fibrin + gelatin microsphere gel sheet retained iPIC within the gel sheet and was found to have no leakage of cells.

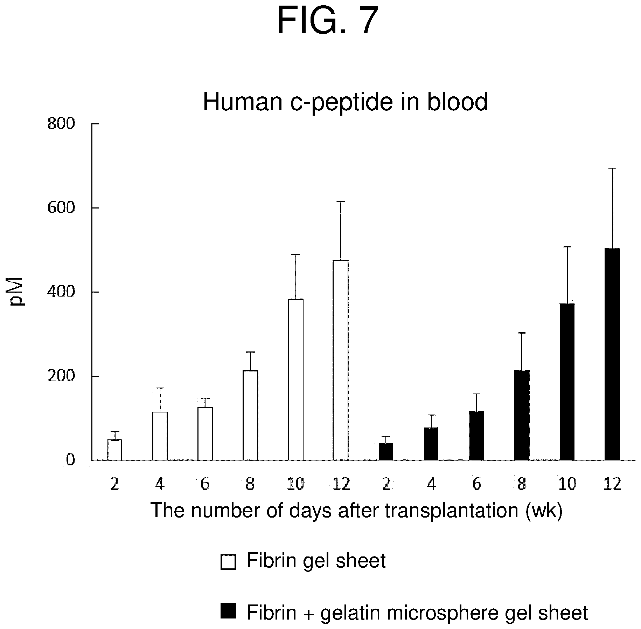

Figure 7 shows results of measuring a human c-peptide concentration in blood over time in the nude rat in which the iPIC-embedded fibrin gel sheet or the iPIC-embedded fibrin + gelatin microsphere gel sheet was subcutaneously transplanted. No significant difference was found in human c-peptide level in blood between the fibrin gel sheet and the fibrin + gelatin microsphere gel sheet, indicating that use of the gelatin microsphere in the sheet has no negative effect on the human c-peptide secretion of iPIC.

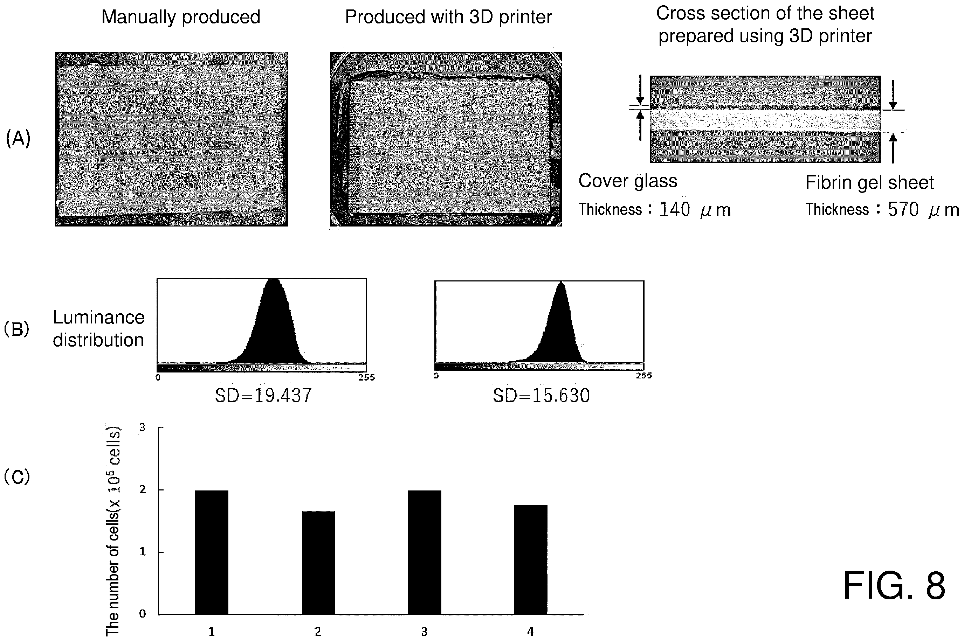

Figure 8 shows the large animal/human-sized iPIC-embedded fibrin + gelatin microsphere gel sheet prepared manually by pipette injection or using a 3D printer. Both the methods are capable of preparing the large animal/human-sized fibrin + gelatin microsphere gel sheet, and the resulting gel sheet retained iPIC within the gel and was found to have no leakage of cells (Figure 8(A)).

Standard deviation was calculated by image-analyzing the luminance distributions of the large animal/human-sized iPIC-embedded fibrin + gelatin microsphere gel sheets prepared manually by pipette injection or using a 3D printer, and was consequently 19.437 and 15.630, respectively (Figure 8(B)). This result indicated that use of the 3D printer for preparation can achieve more uniform application of the fibrin + gelatin microsphere gel. A round shape of 12 mm in diameter was hollowed out at any four locations in the fibrin + gelatin microsphere gel sheet prepared using a 3D printer, and the numbers of cells embedded therein were measured. As a result, almost the same numbers of cells were confirmed (Figure 8(C)). This result indicated that use of the 3D printer can achieve preparation of a fibrin + gelatin microsphere gel sheet in which cells are uniformly distributed and embedded.

### Example 4 Preparation of large animal/human-sized fibrin gel sheet (production method 2-2_fibrin + collagen gel sheet)

### <Method>

### Confirmation of retention of cell dispersibility

A fibrinogen solution (80 mg/mL) and a collagen solution (3 w/v%) were mixed at a ratio of 3:1 to prepare a fibrinogen + collagen solution. iPIC was suspended in the fibrinogen + collagen solution and then packed in a syringe. The syringe was kept vertical with its nozzle facing downward, and left standing for 0, 10, 20, and 30 minutes. Then, an aliquot of an ejected solution was collected, and the number (concentration) of cells was measured to evaluate the fibrinogen + collagen solution for its function of retaining cell dispersibility.

### In vivo test

A fibrinogen solution (80 mg/mL) and a collagen solution (3 w/v%) were mixed at a ratio of 3:1 to prepare a fibrinogen + collagen solution. 3 × 10⁶ cells of iPIC were collected into a 1.5 mL tube and centrifuged. Then, a medium supernatant was removed, and the cells were suspended by the addition of 25 µL of the fibrinogen + collagen solution. The fibrinogen + collagen solution of the iPIC suspended therein was thinly applied onto a glycolic acid/lactate polyester (90/10, poly(glycolideco-L-lactide)) mesh impregnated with 12.5 µL of a thrombin solution (125 U/mL) and then 25 µL of a thrombin solution was wholly applied, and the resultant was left standing for 5 minutes for gelation to prepare an iPIC-embedded thin-layer fibrin + collagen gel sheet.

The iPIC-embedded fibrin + gelatin microsphere gel sheet described in *"In vivo* test" of Example 3 or the iPIC-embedded fibrin + collagen gel sheet was subcutaneously transplanted to a nude rat under anesthesia. After transplantation, blood was collected from the tail vein, and plasma was separated, followed by the measurement of a human c-peptide concentration by

### ELISA.

### Preparation of large animal/human-sized gel sheet

75 × 10⁶ cells of iPIC were collected into a 15 mL tube and centrifuged. Then, a medium supernatant was removed, and the cells were suspended by the addition of 600 µL of the fibrinogen + collagen solution. The fibrinogen + collagen solution of the iPIC suspended therein was thinly applied onto a poly(glycolide-co-L-lactide), size: 40 × 60 mm) mesh impregnated with 300 µL of a thrombin solution (125 U/mL) by using a 3D printer and then 600 µL of a thrombin solution was wholly applied, and the resultant was left standing for 5 minutes for gelation to prepare an iPIC-embedded thin-layer fibrin + collagen gel sheet.

### <Results>

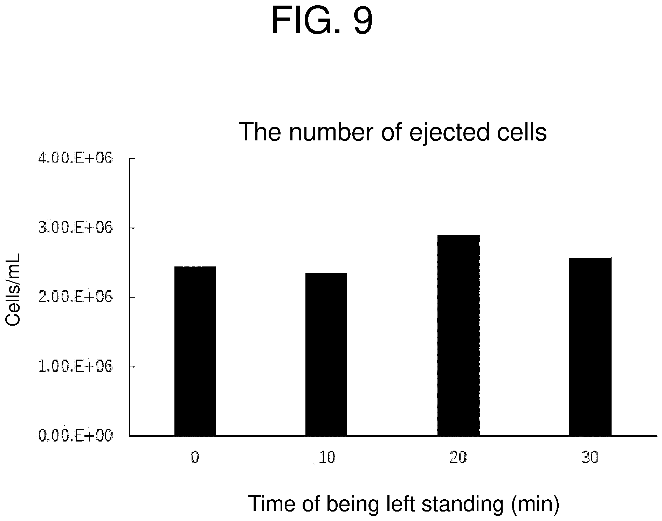

Figure 9 shows results of measuring the number (concentration) of cells in the fibrinogen + collagen solution ejected from a syringe. The cell concentration in the ejected solution was almost constant irrespective of the time of being left standing, indicating that almost a constant number of cells was ejected without precipitating iPIC in spite of keeping the syringe vertical. These results indicated that use of the fibrinogen + collagen solution can maintain the dispersed state of cells.

The produced iPIC-embedded thin-layer fibrinogen + collagen sheet retained iPIC within the gel sheet and was found to have no leakage of cells.

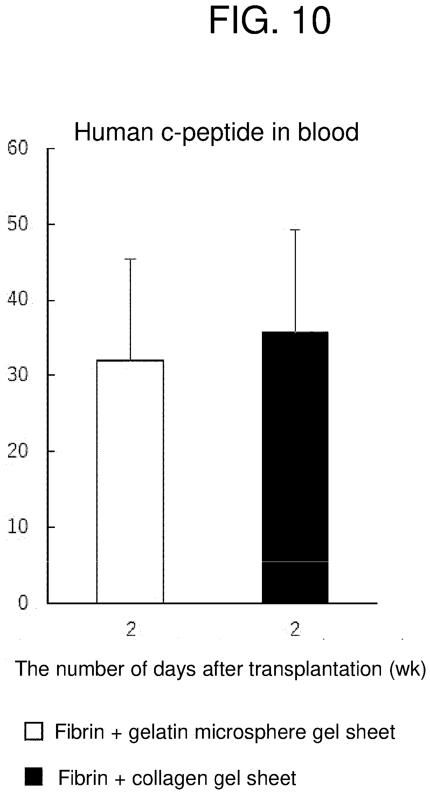

Figure 10 shows results of measuring a human c-peptide concentration in blood over time in the nude rat in which the iPIC-embedded fibrin + gelatin microsphere gel sheet or the iPIC-embedded fibrin + collagen gel sheet was subcutaneously transplanted. No significant difference was found in human c-peptide level in blood between the fibrin + gelatin microsphere gel sheet and the fibrin + collagen gel sheet, indicating that both the gel sheets had equivalent performance.

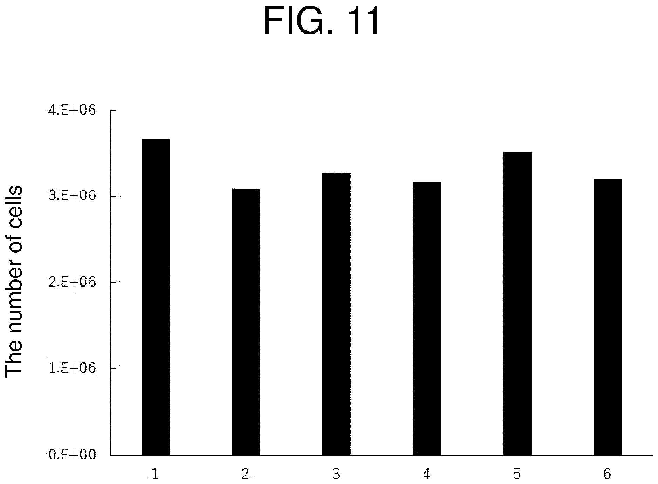

The large animal/human-sized fibrin + collagen sheet was able to be prepared using a 3D printer, and retained iPIC within the gel and was found to have no leakage of cells. A round shape of 12 mm in diameter was hollowed out at any six locations in the prepared large animal/human-sized fibrin + collagen gel sheet, and the numbers of cells embedded therein were measured. As a result, almost the same numbers of cells were confirmed (Figure 11). This result indicated that use of the fibrinogen + collagen solution in the suspension of iPIC and the 3D printer in application can achieve preparation of a fibrin + collagen gel sheet in which cells are uniformly distributed and embedded.

## Claims

1. A fibrin gel sheet for cell transplantation, wherein
cells are uniformly dispersed and embedded in the fibrin gel sheet, and
the fibrin gel sheet further comprises collagen in an amount of 0.2 to 2 w/v% and/or gelatin gel particles in an amount of 10 to 30 w/v%, the gelatin gel particles being capable of passing through 100 µm to 1000 µm.

2. The fibrin gel sheet according to claim 1, wherein the fibrin gel sheet has a size of 2.25 cm² or larger in terms of a surface area of one surface and has a thickness of 1 mm or smaller.

3. The fibrin gel sheet according to claim 1, wherein the cells are embedded in an amount of more than 1.5 × 10⁶ cells/cm².

4. The fibrin gel sheet according to claim 1, wherein the cells are in a spheroid form.

5. The fibrin gel sheet according to claim 1, wherein the cells are iPS cell-derived pancreatic islet cells.

6. The fibrin gel sheet according to claim 1, further comprising a support.

7. A method for producing a fibrin gel sheet for cell transplantation in which cells are uniformly dispersed and embedded in the fibrin gel sheet, comprising the step of
allowing thrombin to act on a fibrinogen solution of the cells suspended therein, and shaping a sheet, followed by gelation, wherein
the fibrinogen solution of the cells suspended therein further comprises collagen in an amount of 0.2 to 2 w/v% and/or gelatin gel particles in an amount of 10 to 30 w/v%, the gelatin gel particles being capable of passing through 100 µm to 1000 µm.

8. The production method according to claim 7, wherein the step of gelation comprises allowing thrombin to act on the fibrinogen solution of the cells suspended therein, and shaping a sheet that has a size of 2.25 cm² or larger in terms of a surface area of one surface and has a thickness of 1 mm or smaller, followed by gelation.

9. The production method according to claim 7, wherein the cells are suspended in the fibrinogen solution in an amount that attains more than 1.5 × 10⁶ cells/cm² in the fibrin gel sheet.

10. The production method according to claim 7, wherein the cells are in a spheroid form.

11. The production method according to claim 7, wherein the cells are iPS cell-derived pancreatic islet cells.

12. The production method according to claim 7, wherein the sheet is shaped by applying the solution onto a support.

13. The production method according to claim 12, wherein the solution is applied onto the support by bioprint.

14. The production method according to claim 12, wherein the support comprises the thrombin.

15. A fibrin gel sheet laminate for cell transplantation, wherein a plurality of fibrin gel sheets according to claim 1 are laminated.
